# EUROPEAN PATENT SPECIFICATION

(11) **EP 2 865 748 B1**
(45) Date of publication and mention of the grant of the patent: **26.09.2018**
(21) Application number: 13796869.9
(22) Date of filing: 29.05.2013
(51) Int. Cl.: C12N 1/20, C12N 9/52, A61K 38/48, A61P 19/04, C12P 1/04

(54) **CULTURE MEDIUM FOR BACTERIA OF THE GENUS CLOSTRIDIUM WITHOUT COMPONENTS OF ANIMAL ORIGIN, AND METHOD FOR PRODUCING A SUPERNATANT CONTAINING ONE OR MORE PROTEASES WITH COLAGENOLYTIC AND GELATINOLYTIC ACTIVITY**
KULTURMEDIUM FÜR BAKTERIEN DER GATTUNG CLOSTRIDIUM KOMPONENTEN OHNE KOMPONENTEN TIERISCHEN URSPRUNGS UND VERFAHREN ZUR HERSTELLUNG EINES ÜBERSTANDS MIT EINER ODER MEHREREN PROTEASEN MIT KOLLAGENOLYTISCHER UND GELATINOLYTISCHER AKTIVITÄT
MILIEU DE CULTURE POUR BACTÉRIES DU GENRE CLOSTRIDIUM EXEMPT DE CONSTITUANTS D'ORIGINE ANIMAL ET PROCÉDÉ DE PRODUCTION D'UN SURNAGEANT CONTENANT UNE OU PLUSIEURS PROTÉASES À ACTIVITÉ COLLAGÉNOLYTIQUE ET GÉLATINOLYTIQUE

(30) Priority: 31.05.2012 BR 102012013110
(43) Date of publication of application: 29.04.2015
(73) Proprietor: Cristália Produtos Químicos Farmacêuticos LTDA., 13970-000 Itapira (BR)
(72) Inventor: ALEGRIA, Marcos Castanheira, CEP: 13970-000 Itapira SP (BR); FARDELONE, Lucidio Cristóvão, CEP: 13970-000 Itapira SP (BR); DELALANA, Marina Baiochi Riboldi, CEP: 13970-000 Itapira SP (BR); THIEMANN, Josef Ernst, CEP: 13970-000 Itapira SP (BR); ASTOLFI FILHO, Spartaco, CEP: 13970-000 Itapira SP (BR); MOREIRA, Roberto Carlos Debom, CEP: 13970-000 Itapira SP (BR); PACHECO, Ogari de Castro, CEP:13970-000 Itapira SP (BR)
(74) Representative: Alves Moreira, Pedro
(86) International application number: PCT/BR2013/000192
(87) International publication number: WO 2013/177647

(56) References cited:
- EP-A1- 2 133 415
- EP-A1- 2 133 415
- WO-A1-98/54296
- WO-A1-2012/125948
- WO-A2-2005/035749
- CN-A- 101 597 582
- CN-A- 102 994 387
- US-A1- 2005 069 562
- FANG A ET AL.: 'Production of Clostridium difficile toxin in a medium totally free of both animal and dairy proteins or digests' PNAS vol. 106, no. 32, 2009, pages 13225 - 13229, XP055095937

## Description

### Field of application

The present invention is related to the field of microbiology and biotechnology. Particularly, this invention is related to a culture medium for bacteria of the genus *Clostridium,* animal product-free, and a process for producing supernatant comprising one or more protease with collagenolytic and gelatinolytic activity.

### Background of the Invention

Collagenase (clostridiopeptidase A, EC 3.4.24.3) is a proteolytic enzyme capable of hydrolyzing collagen proteins, both in their native and denatured (gelatins) form. No other enzyme is capable of cleaving native collagen, due to its distinct amino acid composition (presence of a numerous amount of imino acids, represented by tripeptide Gly-Pro-X, where X is, frequently, proline or hydroxyproline), and its single macrostructure (complex triple-helix structure).

Collagenase, isolated or as the main component of compositions comprising other proteolytic enzymes, has been widely used since the 70's in the treatment of several diseases and pathological conditions. All diseases and conditions in which collagenase is administered are associated with the excessive deposit of collagen and the erratic accumulation of fibrous tissue rich in collagen. Such diseases and conditions are denominated collagen-mediated diseases, and the local application of compositions containing collagenase is one of the possible treatments serving as an alternative for surgical intervention. The use of compositions containing collagenase for the treatment of necrotic tissue debridement (burns, skin lesions, etc.) has also been approved by regulatory agencies, in which such administration has shown surprising results on improving scarification processes.

Some therapeutic applications of compositions containing collagenase are: burn treatment, with a concomitant beneficial effect over tissue regeneration; enzymatic debridement of wounds; infected wounds and ulcer treatment (of skins and decubitus ulcers); intervertebral disc hernia treatment; selective lysis of collagen fibers of the eye's vitreous body; Dupuytren's disease treatment; Peyronie's disease treatment; adhesive capsulitis treatment; lateral epicondylitis treatment, Carpal Tunnel Syndrome and plantar fasciitis; increase of regeneration of damaged nerves; also being widely used in medical specialties such as plastic surgery and in several dermatological and aesthetic procedures, for example, in the treatment of cellulites and mammal scars, such as acne, keloid and other hypertrophic scars through intralesional injection of purified collagenase.

Other studies have shown the use of compositions containing collagenase in rheumatoid arthritis, metastasis, angiogenesis and cirrhosis.

Collagenases have also been described as important tools in specific organ transplantation i.e. isolating of pancreatic islets of Langerhans, microvascular endothelial cells, hepatocytes and chondrocytes (WO9824889). Tissue dissociation mediated by collagenolytic enzyme is a critical step in several cell isolation procedures.

The collagenases used in the majority of the therapeutic applications described above are extracted from the supernatant culture, purified or not, of bacterial cultures, most specifically of *Clostridium histolyticum* cultures. *Clostridium histolyticum* is an anaerobic, gram-positive and spore-forming cylindrical bacterium. The collagenolytic activity of the extracellular enzymes of such bacteria has been known for over 50 years, when it was isolated for the first time an extracellular enzyme capable of digesting cattle Achilles tendons.

Currently, it is of common knowledge that several microorganism species when cultured in specified conditions are capable of producing collagenase. However, *Clostridium sp,* most specifically *Clostridium histolyticum,* is still the main source of collagenolytic and gelatinolytic enzymes for therapeutic application due to its higher enzymatic productivity and activity against several collagen forms and its peptides.

Other collagenase sources for therapeutic use include mammal cells, crustaceans (crabs, shrimp), fungi and other bacteria (*Streptomyces, Pseudomonas and Vibrio*).

The ability of *Clostridium* collagenases to digest several kinds of collagen (type I, II, III, VII and X) and gelatin is the major factor that differentiates these proteases from other collagenase sources. Aforesaid clostridial enzymes not only present higher collagenolytic activity when compared to vertebrate collagenases but it also presents an optimum pH for its activity within human physiological pH range.

Studies from late 1950s to mid-1980s showed the existence of several types of collagenases produced by *C. histolyticum* and the specificity and stability of these fractions were gradually characterized. Bond and Van Wart (1984; Biochemistry, 23: 3077-3085) isolated six different enzymes from commercial collagenase preparations with molecular weights varying of 68 kDa to 130 kDa having isoelectric points between pH 5.5 and 6.5; further, classifying them in two classes according to substrates specificity. Class I - presenting higher activity against high molecular weight collagens (native collagen: intact); Class II - demonstrates a preference for low molecular weight collagen fragments (denatured collagen: gelatins). These activities seem to be complementary, and there is evidence of its synergistic action on native collagen.

In addition to the several class I and II collagenase isoforms, traditional preparations of collagenase, based on the supernatant of *Clostridium histolyticum* cultures, contain over 30 different enzymes. The primary constituent of the culture supernatant are the collagenases (classes I and II), however, other important proteases are also present, some such are: neutral proteases i.e.: gelatinases, clostripains (clostridiopeptidase B, EC 3.4.22.8), trypsins, elastases and aminopeptidases. Non-proteolytic enzymes including: galactosidase, acetylglucosaminidase, fucosidase, phospholipase, neuraminidase (or sialidase) and hyaluronidase can also be found. Of the aforementioned proteolytic and non-proteolytic enzymes, sialidase, hyaluronidase and collagenase are deeply involved in the degradation process of extracellular matrix components. The presence of this "enzymatic combination" is required to obtain compositions suitable to be used as enzymatic debridement agent for skin lesion and organ transplant (i.e. isolation of pancreatic islets).

Furthermore, either purified or recombinant collagenase alone is inefficient in tissue dissociation due to incomplete hydrolysis of all collagen polypeptides and to its limited activity when in high concentrations of non-collagen proteins and other macromolecules found in the extracellular matrix. For such reason, the most used collagenase composition for tissue dissociation and debridement is a crude or partially purified preparation (obtained by few purification steps). Since the presence of enzymes which act on native collagen and reticular fibers in addition to enzymes responsible for the hydrolysis of other proteins, polysaccharides and lipids in the extracellular matrix of connective and epithelial tissue are required. Although such preparation does not require complex purification steps, the difficulty lies in the production process, due to the different expression patterns of the proteolytic enzymes required in the final composition.

On the other hand, the use of compositions of collagenase for the treatment of collagen-mediated diseases require high purity of the preparations, due to the need of a specific action on digestion of collagen; such treatments are administered through local injections. In these cases, the processes of purification of the supernatant of *C. histolyticum* cultures are very long, including numerous steps with different strategies of purification.

In general, the collagenase composition purity is directly related to the therapeutic application intent. However, regardless of the therapeutic application, a defined process for culturing *Clostridium histolyticum* is essential to direct the production in order to obtain the proteases of interest in the supernatant, purified or not.

The enzymatic activity and the concentration of proteases in *C. histolyticum* cultures supernatant vary according to the bacterial strain, culture medium, culture conditions, culture age, cellular density, among other variables. The intrinsic variability of this type of biotechnological process implies in exhaustive quality control analysis of the produced batches, increasing production costs. Moreover, the loss of efficacy of traditional collagenase along time, regardless of the storage conditions, is strongly related to the enzymatic composition of the supernatant. For such reason, defined culture conditions which direct the production of the desired enzymes collagenolytic and gelatinolytic enzymes, in special collagenases are required.

It is well known that the production of extracellular proteases in microorganisms is strongly influenced by components of the medium, especially by carbon and nitrogen sources, and physical factors such as, for instance, pH, temperature, inoculum volume, rate of orbital agitation and incubation time. There is no pre-defined media for specific proteases, each microorganism has its own physical-chemical and nutritional idiosyncrasies for enzymatic expression and secretion. Proteins and peptides are the main source of carbon and nitrogen in *Clostridium* culture, being required for protein expression as well as growth. Therefore, brain-heart infusion (BHI), tryptose, peptone proteose, meat extract, casein and gelatin are commonly found in *C. histolyticum* culture. Not only, inorganic salts, vitamins, glucose, yeast extract, sodium thioglycolate and ferrous sulphate are also found in different concentrations in such culture media.

Since the thirties, several authors stated that animal origin peptones are essential for *C. histolyticum* growth and enzymatic production.

Later, in the 1950s, MacLennan et al. (1953; J. Clin. Invest., 32: 1317) evaluated how several carbon and nitrogen sources (phytones, peptones, collagen and it's derivatives, and glucose), as well as vitamin and inorganic salts concentration, pH and Fe⁺⁺ influence on *C. histolyticum* growth and collagenolytic and gelatinolytic enzyme expression (several strains). The results presented in this study have been considered the "conventional culture medium" for *C. histolyticum* collagenase production for several decades. Ferrous salt and animal origin peptone have been defined as crucial for maximum collagenase yield. Commonly described as examples of animal origin peptones are: bacto-peptone, peptone proteose, hydrolyzed casein, casein tryptic hydrolysate, among others. Combinations of these components with vegetable peptones have also been described.

For example, Bergman et al. (1961; Journal of Bacteriology, 82: 5829) obtained good results culturing *C. histolyticum* in a culture medium which contains peptone proteose, casein hydrolysate, tryptic hydrolysates of soybean and vitamins. It has been demonstrated that, in the absence of inorganic salts, the collagenase productivity is equal to or greater than the one presented by MacLennan *et al.* (1953). What occurs, however, is a decrease in the production of azocaseins, which is an interesting fact, since it facilitates later purification procedures (called PTV culture medium).

Besides the importance of animal origin peptones for the growth and enzymatic production in cultures of *C. histolyticum,* the influence of other components in the culture medium has also been investigated in order to define metabolic patterns for this species, thus improving culturing conditions.

Mead (1971; J. Gen. Microbiol., 67: 47) describes the pattern of amino acids intake in several species of the genus *Clostridium* during its growth, classifying them into groups according to their nutritional needs. In the afore mentioned study, *Clostridium histolyticum* is considered capable of fermenting amino acids, and is sub-classified in the so called Group II (*C. botulinum; C. histolyticum; C. cochlearium; C. subterminale*). Even though serine, glutamine, glycine and valine are the main amino acids used by these species, the addition of theses in culture medium produces no or little effect on the bacterial growth. However, by substituting partial casein hydrolysate by casamino acids significantly increases the observed growth rate (culture medium used: vitamins, inorganic salts, tryptophans, cysteine hydrochloride, casamino acids - 2 g/L or casein hydrolysate - 3% w/v and meat extract). Unfortunately, the author did not analyze the protease production.

More frequent than the amino acids intake, glucose intake as energy source for several microorganisms has been well documented. Particularly, for *C. histolyticum* the addition of glucose (and other sugars) has been correlated with a decrease in bacterial growth rate (Mead, 1971). Thus despite being initially classified as saccharolytic species, due to their ability of metabolizing carbohydrates as energy source, several strains of *C. histolyticum* inefficiently simple sugars such as glucose, maltose, among others. Moreover, a decrease in growth rate is even more prominent when culturing *C. histolyticum* in media containing glucose and in aerobiotic conditions, suggesting the existence of two inhibiting factors: sugars and oxygen/air.

Not only does glucose negatively influence bacterial growth, it has also been reported that glucose containing medium decreases protease expression in *Clostridium* cultures (*C. difficile,* for example). This observation suggests that there is a relation between toxin expression repression and rapidly consumable carbon sources.

As discussed previously, bacterial growth rate and protein expression by species within the genus *Clostridium* are affected by nutritional requirements as well as the maintenance of an anaerobic environment. *C. histolyticum* tolerates few amounts of oxygen, characteristic common in some anaerobes and also other species of the genus, for such reason it can be considered an aero tolerant microorganism. Therefore, minimal conditions of anaerobiosis must be secured for culturing *C. histolyticum* for protease production with high collagenolytic and gelatinolytic activity.

Additionally, reducing agents are also considered critital in anaerobe liquid culture. Among the most frequently used agents are: digest of meat, glucose, sodium thioglycolate, cysteine, iron salts, metallic iron or a mixture of both.

To this date, it remains unknown which components, how they influence and what are their effects on C. *histolyticum* growth and protease secretion. However, it is known that, from studies carried out, animal origin ingredients, in particular peptone, positively influence *Clostridium histolyticum* growth and protease expression, among them collagenases.

The conventional *C. histolyticum* culturing processes present several disadvantages, such as: low yield, low reproducibility and incomplete separation of impurities. Furthermore, one of the major problems such process is the predominant use of animal origin components in the production of proteases with collagenolytic and gelatinolytic activity for therapeutic use in humans.

The presence of animal origin components for obtaining proteases with collagenolytic and gelatinolytic activity in *C. histolyticum* culture for therapeutic use in humans offers, undesirably, potential risk of infections and anaphylactic reactions. A well-known example of disease caused due to interspecific horizontal transmission of pathogens is the bovine spongiform encephalopathy (prion disease; "Mad Cow disease"). Its transmission from the original host (bovine) to man was first recorded in 1993, it was caused due to human contact with physiological fluids and by eating infected animal meat.

In the last decade, regulatory agencies have been encouraging the pharmaceutical industry to use bacterial culture media comprising only non-animal derived products. However, care must be taken when considering non-animal derived compounds since these are often processed using animal derived enzymes (protein and carbohydrates are digested to generate peptones and sugars which are used in the medium). When animal-free compounds are treated with animal derived enzymes, even in very small fraction, the final product cannot be classified as animal-free. The following patents and papers state different animal-free culture media for *Clostridium* culture: WO9854296 (1998, Chiron), WO0105997 (2000, Massachusetts Institute of Technology), WO2005035749 (2004, Allergan), Busta & Schroder (1971, Effect of soy proteins on the growth of Clostridium perfringens. Appl. Microbiol, 22(2), 177-183; Demain et al. (2007, Tetanus toxin production in soy-based medium: nutritional studies and scale-up into small fermentors. Lett. Appl. Microbiol., 45(6), 635-638); Fang et al. (2009, Production of Clostridium difficile toxin in a medium totally free of both animal and dairy proteins or digests. PNAS, vol. 106: 13225-13229).

It has already been investigated and demonstrated that components obtained from soybean and yeast extracts can be used to replace animal derived peptones in some species of the genus *Clostridium* such as *C. sporogenes, C. tetanus, C. botulinicum.* Such extracts are capable of sustaining growth rate as well as protease expression and secretion

In the particular case of cultivating *C. histolyticum,* especially for proteases with collagenolytic and gelatinolytic activity production, the state of the art reveals next to nothing about the composition of animal product-free culture media capable of stimulating the production of these proteases in a way at least equivalent to the traditional media containing animal origin peptones.

The culture media described so far for the *C. histolyticum* presents disadvantages such as: presence of animal components, high cost associated with its complex composition, low collagenase expression and long culturing periods required to obtain a supernatant with collagenolytic and gelatinolytic activity appropriate for industrial production with therapeutic purposes.

Attempts for obtaining an animal product-free culture medium that would be appropriate for *C. histolyticum* growth and collagenase production are reported below.

The document WO2007089851 (2007; Auxilium) describes *C. histolyticum* culture conditions in order to obtain collagenases. Although it describes the production of collagenases by *C. histolyticum* in an animal product-free culture medium, it still emphasizes that in order to produce collagenases with higher reproducibility, a culture medium comprising peptone protease of animal origin is preferred. Only the animal origin culture medium was capable of producing the collagenases in adequate proportion to maximize its synergistic activity, resulting in a therapeutic benefit. Other than peptones (vegetable and/or animal), the culture medium described above may contain amino acids (glutamine, tryptophan and asparagine), yeast extract, inorganic salts, glucose and vitamins.

The document US20100086971 (2009; Roche) describes a culture medium for *C. histolyticum* which includes plant derived peptone. However, an animal derived additive - fish gelatin - is considered crucial for most of the supernatant collagenolytic activity in these cultures. It is emphasized that the liquid composition containing only vegetable and fish gelatin peptones can withstand greater bacterial growth and more collagenolytic activity when compared to the standard culture medium comprising animal origin peptone.

Thus, until the present moment, no culture medium completely animal product-free has been capable of supporting an adequate industrial *C. histolyticum* collagenases production as demonstrated through traditional culture media containing animal peptones.

For such reason, there is a clear need for developing animal product-free culture media as well as processes for *C. histolyticum* proteases with collagenolytic and gelatinolytic activity production.

Therefore, the present invention provides a culture medium for bacteria of the genus *Clostridium,* preferably *C. histolyticum,* animal product-free and a process for the production of supernatant comprising one or more proteases with collagenolytic and gelatinolytic activity adequate for industrial collagenolytic and gelatinolytic enzymes production, in particular collagenases, for therapeutic purposes.

### Summarized Description of the Invention

The present invention refers to an animal product-free culture medium for bacteria of the genus *Clostridium* comprising water, non-animal origin peptone or its derivatives, yeast extract and the amino acids cysteine and arginine, or pharmaceutically acceptable salts thereof. Preferably, the bacteria belonging to *Clostridium histolyticum* strain.

The culture medium of the present invention can additionally comprises one or more additives selected from the group consisting of agent for adjustment pH in the range between 7 and 8, reducing agent, inorganic salts and vitamins.

The non-animal origin peptone may be vegetable or yeast peptone. Preferably, the non-animal origin peptone is a vegetable peptone selected from the group consisting of soybean, cotton, wheat, sunflower, rice, peanut, fava bean, peas, potato, corn or mixtures thereof. Most preferably, the vegetable peptone is a soybean peptone.

The culture medium of the present invention is characterized by the fact that the non-animal origin peptone concentration ranges from 0.5% to 5% w/v; yeast extract concentration ranges from 0.5% to 5% w/v; cysteine concentration, or its pharmaceutically acceptable salts, ranges from 0.01% to 0.1% w/v; and arginine concentration, or its pharmaceutically acceptable salts, ranges from 0.1% to 1% w/v. Preferably, the non-animal origin peptone concentration ranges from 1% to 4% w/v; the yeast extract concentration ranges from 1% to 4% w/v; the cysteine concentration, or its pharmaceutically acceptable salts, ranges from 0.03% to 0.07% w/v; and the arginine concentration, or its pharmaceutically acceptable salts, ranges from 0.25% to 0.7% w/v. Most preferably, the non-animal origin peptone concentration is 3% w/v; the yeast extract concentration is 3% w/v; the cysteine concentration, or its pharmaceutically acceptable salts, is 0.0625% w/v; and the arginine concentration, or its pharmaceutically acceptable salts, is 0.375% w/v.

Differing from the culture media conventionally used for *C. histolyticum* growth comprising at least one animal origin ingredient, the culture media of the present invention are animal product-free and, surprisingly, stimulate proteases with collagenolytic and gelatinolytic activity production, particularly collagenases, being, such as it is, superior to the conventional culture media which contain animal origin peptones.

The present invention also refers to a process for *Clostridium histolyticum* liquid culturing in order to produce a supernatant comprising one or more proteases with collagenolytic and gelatinolytic activity characterized by comprising the steps of: **a)** providing a sterile animal product-free culture medium; **b)** culturing an aliquot of *Clostridium histolyticum* stock culture in the culture medium in step (a), under anaerobic conditions at about 37°C before reaching the stationary growth phase in order to obtain an inoculum; **c)** providing a sterile animal product-free culture medium for *Clostridium* bacteria, according to present invention; **d)** adding to the culture medium in step (c) a volume of inoculum from step (b) equal to or lower than 10% of the final volume of the medium defined in (c); **e)** culturing the *C. histolyticum* obtained in (d) under anaerobic conditions at about 37°C until the stationary growth phase is attained; **f)** removing the cellular material and other particulate matter in the liquid phase of the culture in step (e), producing a supernatant comprising one or more proteases with collagenolytic and gelatinolytic activity. Such process may include one or more additional steps for the purification of one or more proteases with collagenolytic and gelatinolytic activity present in the supernatant obtained in step (f).

The present invention also refers to a supernatant of a *C. histolyticum* liquid culture comprising one or more proteases with collagenolytic and gelatinolytic activity obtained according to the process mentioned above, and pharmaceutical compositions comprising, as active ingredient, the supernatant of *Clostridium histolyticum* liquid culture and pharmaceutically acceptable excipients.

### Brief Description of the illustrations

Aspects of the invention are explained or depicted by the following figures:

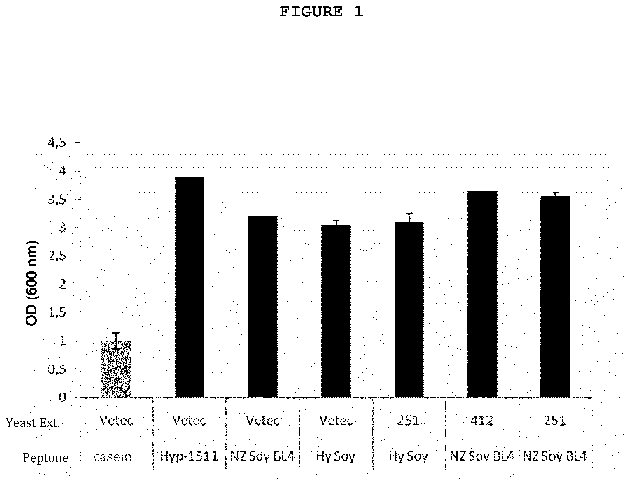

**Figure 1****.** Comparison between the bacterial growth (optical density 600 nm) of *C. histolyticum* (ATCC 21000) in an animal product-free culture medium (3% w/v of vegetable peptone, 3% w/v of yeast extract, 0.0625% w/v of cysteine hydrochloride) that differ relative to the kind of vegetable peptone and yeast extract used. Standard culture medium (comprising casein peptone) was used for comparison.

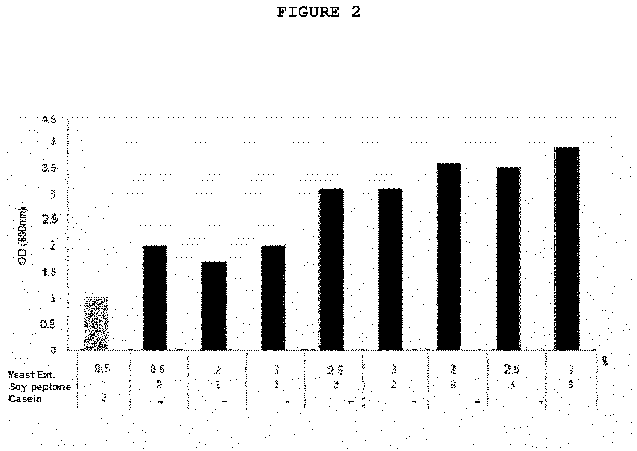

**Figure 2****.** Comparison between the bacterial growth (optical density 600 nm) of *C. histolyticum* (ATCC 21000) in an animal product-free culture medium that differ quantitatively relative to the vegetable peptone component (HY Pep 1511) and yeast extract (YE5114). The animal product-free culture medium contains 0.0625% w/v of cysteine hydrochloride. Standard culture medium (comprising casein peptone) was used for comparison.

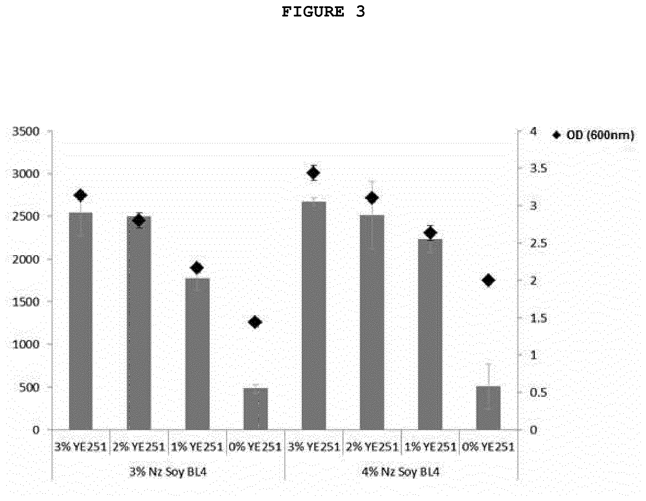

**Figure 3****.** Influence on the growth (OD) and enzymatic activity of different concentrations of the yeast extract (0-3% w/v) in the composition of the animal product-free culture medium for *C. histolyticum* (ATCC 21000) comprising 3% w/v or 4% w/v of soybean peptone and 0.0625% w/v of cysteine hydrochloride.
**Figure 4****.** pH influence on bacterial growth (optical density 600 nm) of *C. histolyticum* (ATCC 21000) in an animal product-free culture medium comprising 3% w/v of soybean peptone (NZ Soy BL4), 3% w/v of yeast extract (YE251) and 0.0625% w/v of cysteine hydrochloride.

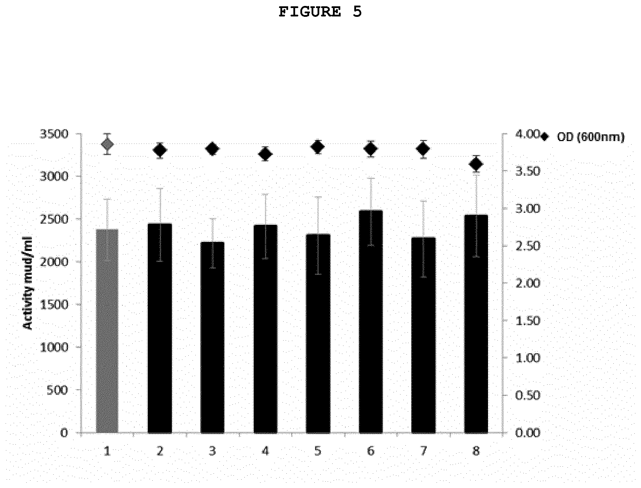

**Figure 5****.** Influence of supplementing the medium with inorganic salts, vitamins and amino acids on the growth and the collagenolytic and gelatinolytic activity of the supernatant obtained from a liquid culture of *C. histolyticum* (ATCC 21000) in an animal product-free culture medium comprising: 3% w/v of soybean peptone (NZ SOY BL4), 3% w/v of yeast extract (YE 251) and 0.0625% w/v of cysteine hydrochloride. The bars (1-8) in the chart refer to:
1 - Ctrl. Medium:
   - NZ-soy BL4 (3%);
   - Yeast extract YE 251 (3%);
   - Cysteine hydrochloride (0.0625%);
2 - Ctrl + Salts*
3 - Ctrl + Vitamins**
4 - Ctrl + Vitamins** + Salts*
5 - Ctrl + Aa***
6 - Ctrl + Aa*** + Vitamins**
7 - Ctrl + Aa*** + Salts*
8 - Ctrl + Aa*** + Vitamins** + Salts*

The vitamins and the salts were tested in the following concentrations:

| **Salts *** | |
|---|---|
| **Components** | **Quantities (mg/ml)** |
| **Potassium phosphate dibasic (K₂HPO₄)** | **1.0** |
| **Potassium phosphate monobasic (KH₂PO₄)** | **1.0** |
| **Magnesium sulphate (MgSO₄.7H₂0)** | **0.4** |
| **Sodium chloride (NaCl)** | **0.02** |
| **Iron sulphate (FeSO₄.7H₂0)** | **0.02** |
| **Manganese sulphate (MnSO₄.4H₂0)** | **0.02** |

| **Vitamins **** | |
|---|---|
| **Biotin** | **0.00625** |
| **Pimelic acid** | **0.02** |
| **Nicotinamide** | **0.02** |
| **Calcium pantothenate** | **0.02** |
| **Folic acid** | **0.02** |
| **Thiamine nitrate** | **0.02** |
| **Riboflavin** | **0.02** |
| **P-Aminobenzoic acid (PABA)** | **0.02** |

| **Amino acids - Aa***** | |
|---|---|
| **Mix 20 Aa** | **1/each** |

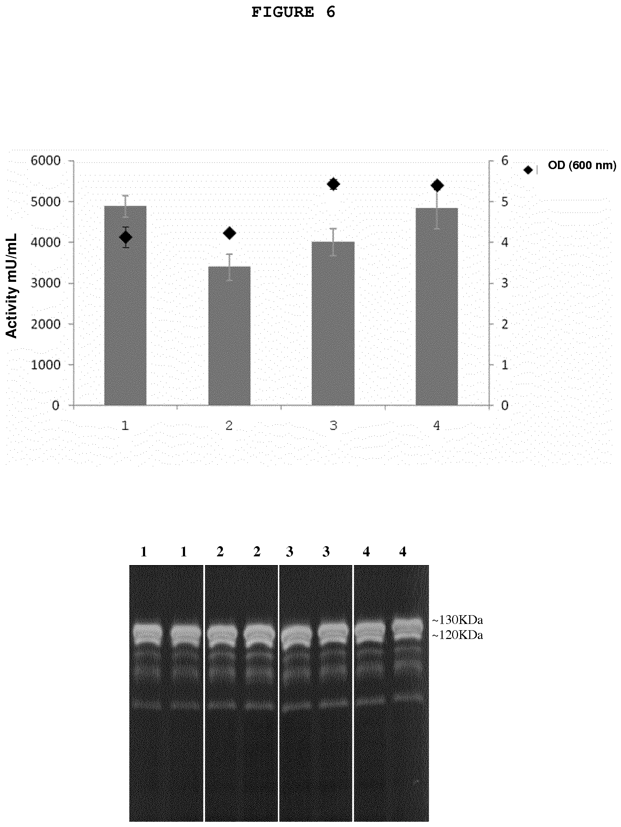

**Figure 6****.** Influence of supplementing the medium with inorganic salts, vitamins and amino acids on the growth and the collagenolytic and gelatinolytic activity in the supernatant obtained from a liquid culture of *C. histolyticum* (ATCC 21000) in an animal product-free culture medium comprising: 30 g/L of soybean peptone (NZ SOY BL4), 30 g/L of yeast extract (YE 251) and the cysteine hydrochloride (0.625 g/L) and arginine hydrochloride (3.75 g/L). Analysis of the enzymatic activity was observed through zymography gel (samples of the supernatant obtained for each culture medium were analyzed in duplicates). The bars (1-4) in the chart refer to:
1 - Ctrl. medium + arginine:
   - vegetable peptone NZ-soy BL4 (30 g/L);
   - Yeast extract YE 251 (30 g/L);
   - Cysteine hydrochloride (0.625 g/L);
   - L-arginine (3.75 g/L).
2 - Ctrl + Vitamins
3 - Ctrl + Phosphates
4 - Ctrl + Vitamins + Phosphates

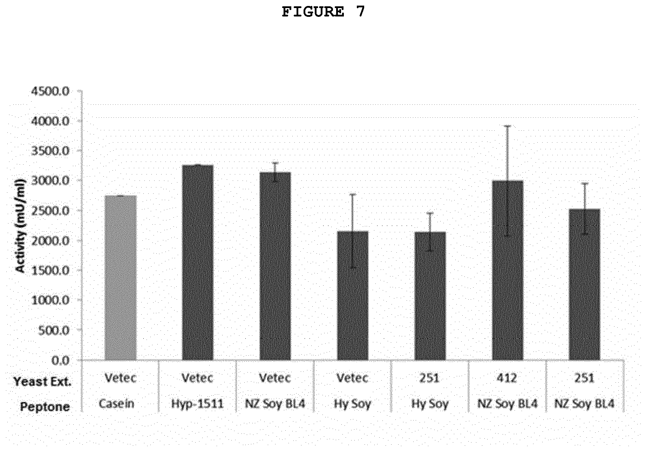

**Figure 7****.** Comparison between the collagenolytic and gelatinolytic activity in the supernatant obtained from *C. histolyticum* (ATCC 21000) liquid culture of animal product-free culture medium (3% w/v of vegetable peptone, 3% w/v yeast extract, 0.0625% w/v of cysteine hydrochloride) that differ relative to the kind of vegetable peptone and yeast extract used. Standard culture medium (comprising casein peptone) was used for comparison.
**Figure 8****.** *C. histolyticum* (ATCC 21000) bacterial growth and collagenolytic and gelatinolytic activity in the supernatant obtained from *C. histolyticum* liquid culture using an animal product-free culture medium (30 g/L of soybean peptone NZ SOY BL4, 30 g/L of YE251 and 0.625 g/L of cysteine) comprising, additionally, arginine, glycin or both. Horizontal axis label:
Ctrl* = NZ-SoyBL4 (30 g/L); Yeast extract YE 412 (30 g/L); Cysteine hydrochloride (0.625 g/L); and
GA** = Glycin + L-arginine-HCl

**Figure 9****.** *C. histolyticum* (ATCC 21000) bacterial growth and collagenolytic and gelatinolytic activity in the supernatant obtained from *C. histolyticum* liquid culture using an animal product-free culture medium (3% w/v of soybean peptone NZ SOY BL4, 3% w/v of YE251 and 0.0625% w/v of cysteine) comprising, additionally, amino acid groups (Groups 1 to 6). Enzymatic activity was analyzed through SDS-PAGE zymography (Groups 1 through 6 represent the culture media comprising the amino acid groups evaluated). The Groups 1-6 and Control in the chart refer to:

| | |
|---|---|
| Group 1: | Glutamate/Proline/Glutamine/Arginine* |
| Group 2: | Aspartate/Asparagine/Lysin/Methionine/Threonine/Isoleucine* |
| Group 3: | Alanine/Valine/Leucine* |
| Group 4: | Serine/Glycin/Cysteine |
| Group 5: | Phenylalanine/Tyrosine/Tryptophan* |
| Group 6: | Histidine* |
| Control: | NZ-Soy BL4 (3%); Yeast extract YE 251 (3%); cysteine hydrochloride (0.0625%). |

| | |
|---|---|
| *Amino acids at 1 mg/ml each. | |

**Figure 10****.** *C. histolyticum* (ATCC 21000) bacterial growth and collagenolytic and gelatinolytic activity in the supernatant obtained from *C. histolyticum* liquid culture using an animal product-free culture medium (3% w/v of soybean peptone NZ SOY BL4, 3% w/v of YE251 and 0.0625% w/v of cysteine) cultured in bioreactor (3 L). Enzymatic activity was analyzed through SDS-PAGE zymography during cultivation.
**Figure 11****.** C. histolyticum (ATCC 21000) bacterial growth and collagenolytic and gelatinolytic activity in the supernatant obtained from C. histolyticum liquid culture using an animal product-free culture medium (3% w/v of soybean peptone NZ SOY BL4, 3% w/v of YE251 and 0.0625% w/v of cysteine and 0.375% w/v of arginine) cultured in a bioreactor (3 L). Enzymatic activity was analyzed through zymography (B) and the proteins were analyzed through SDS-PAGE gel (C) while culturing (the results presented are only after 7 hours of growth).

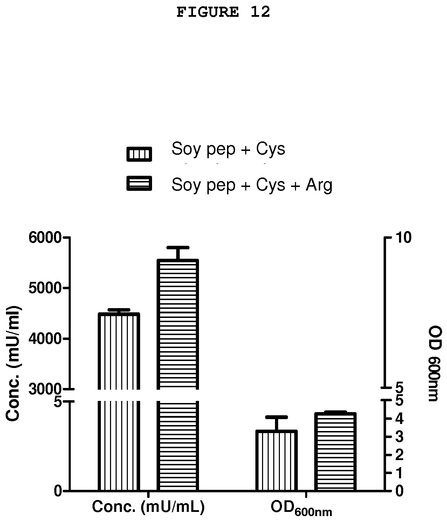

**Figure 12****.** Comparative analysis between growth and collagenolytic and gelatinolytic activity in the supernatant obtained from a *C. histolyticum* (ATCC 21000) liquid culture using an animal product-free culture medium comprising: 3% w/v of soybean peptone NZ SOY BL4, 3% w/v of YE251 and 0.0625% w/v of cysteine; or 3% w/v of soybean peptone NZ SOY BL4, 3% w/v of YE251, 0.0625% w/v of cysteine and 0.375% w/v of arginine. Cultures took place in a bioreactor (3 L). Analyses took place at the end of the culturing process.

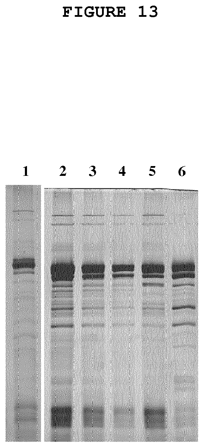

**Figure 13****.** Recovery of proteases with collagenolytic and gelatinolytic activity in the supernatant obtained from a C. *histolyticum* (ATCC 21000) liquid culture using an animal product-free culture medium comprising 3% w/v of soybean peptone NZ SOY BL4, 3% w/v of YE251, 0.0625% w/v of cysteine and 0.375% w/v of arginine. Protein profile was analyzed through SDS PAGE during the process. In the chart below, the columns from 1 to 6 refer to:

| | Activity (U/mL) | Yield (%) |
|---|---|---|
| 1 - Supernatant (4x) | 908.578 | 100 |
| 2 - post-dialysis (4x) | 749.568 | 82.5 |
| 3 - post-dialysis (10x) | | |
| 4 - post-dialysis (25x) | | |
| 5 - post-dialysis (50x) | | |
| 6 - post-lyophilization (25x) | 798.649 | 87.35 |

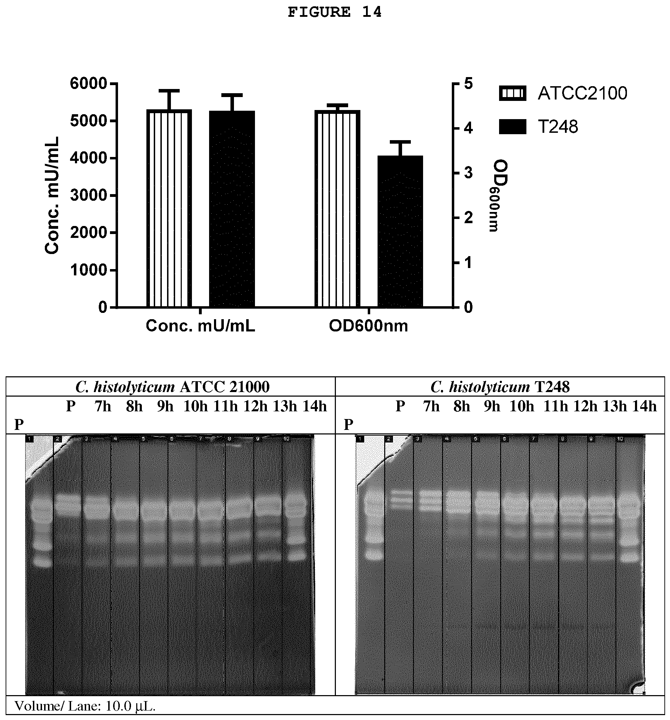

**Figure 14****.** Comparative analysis between growth (DO 600 nm) and collagenolytic and gelatinolytic activity (mU/mL) in the supernatant obtained from liquid culture of *C. histolyticum* ATCC 21000 e T248 in animal product-free culture media comprising: 3% w/v of soybean peptone NZ SOY BL4, 3% w/v of YE251, 0.0625% w/v of cysteine and 0.375% w/v of arginine. Cultures were carried out in bioreactor (3 L). Analyses were performed (14 h) at the end of the culture process.

### Detailed description of the invention

In order to guarantee a better understanding of the invention's scope, without making it a limiting factor, the specific terms of the technology fields related to the present invention are defined as follows.

*"Proteases"* (proteinases, peptidases or proteolytic enzymes, EC 3.4), according to present invention, are enzymes that cleave peptide bonds between the protein amino acids. "collagenases" of *Clostridium histolyticum* as type I and type II accordingly to the description supplied by Bond M. D., van Wart, H. E., 1987, Biochemistry 23:3077-3085 and Bond, M. D., van Wart, H. E., 1984, Biochemistry, 23: 3085.

*"Collagen proteins"* or *"collagen"* refer to the main structural proteins present in the skin and bones of most animals which are important extracellular matrix components. Collagen molecules consist of 3 chains of individual polypeptide chains (alpha chains) folded in a triple helix configuration that is stabilized by hydrogen bonds.

"Gelatin" refers to a partially hydrolyzed form of collagen.

"Collagenolytic and gelatinolytic activity" refers to the measure of collagen and/or gelatin degradation within a specified period of time. Protease collagenolytic and gelatinolytic activity is commonly verified by the protein's ability to cleave and the degradation rate when in the presence of collagen and gelatins. It is usually expressed in units/mL, units/L, units/mg of enzyme or units/g of enzyme. Collagenolytic and gelatinolytic activity quantification can be determined through a variety of methodologies such as viscometric analysis, colorimetric (ninhydrin; Moore & Stein, JBC, 176, 367, 1948) or fluorimetric reactions (collagen and gelatin substrates marked with fluorescence; EnzChek®) among others. In the case in which the activity is quantified following a colorimetric protocol with ninhydrin, the activity is measured by the enzymatic hydrolysis of a synthetic substrate (Carbobenzoxy-Gly-Pro-Gly-Gly-Pro-Ala).

*"Bacterial growth"* is defined as the division of a bacterial cell in two identical daughter cells during a process called binary fission. This way, the bacterial population duplication occurs at each cell division. However, if the number of viable cells exceeds the average, the bacterial population will present exponential growth. Exponential bacterial growth in a culture can be monitored through well-known methods, such as, direct bacterial cell count (i.e. microscopy, flow cytometry), determining biomass value (milligrams, grams, kilos or tons), colony count, optical density (measured in spectrophotometer, wavelength of about 600nm), monitoring nutrient consumption, among others. Bacterial growth can be characterized by four different phases: "*lag phase*", "*exponential or log phase*", "*stationary phase*" and "*decline or death phase*".

It is of common knowledge that during the "*lag phase*", the bacteria are adapting to the presented growth conditions. It is a period when cells are entering a maturity state, not yet capable of dividing themselves. The "*exponential or log phase*" is a period characterized by cell duplication. If growth is not a restraint, cell duplication continues at a steady rate, so both, the number of cells and growth rate, duplicate in each period of time. The exponential growth phase cannot endure indefinitely since the growth medium has nutritional restraints and accumulation of metabolites which are often toxic and are produced by bacterial cells undergoing division. During the "stationary phase", growth rate decreases due to nutritional restraints and accumulation of toxic metabolites. This phase is characterized by resource depletion in the culture medium. In the "*decline or death phase*", bacteria deplete completely the remaining nutrients still available in the culture medium and die.

"*Pre-inoculum*" is defined as a suspension of microorganisms obtained from a stock culture that will be used for continued growth of the microorganism for producing of an inoculum.

"*Inoculum*" is a microorganism suspension with a given specific concentration that is to be used for continued growth and/or fermentation on a larger scale (greater volume of culture medium) than the initial one.

"*Anaerobic condition*" and "anaerobiosis" is defined as the maintenance of a substantially oxygen-free culture condition.

"*Fresh culture medium*" refers to any culture medium for microorganism growth or fermentation that has not been previously used, or, as a medium containing integrally all of its components.

"*Freezing medium*" characterizes a composition capable of maintaining a microorganism's viability after being subjected to freezing or lyophilization and storage and, thus, its growth and fermentation capacity after the storage period.

The storage of the microorganism on the freezing medium can be performed on temperatures that are suitable for its freezing maintenance. Moreover, it is also possible to lyophilize the microorganism in the freezing medium before storage. In the present invention, the freezing medium is an animal product-free and contains, in addition, a cryopreservation agent.

"*Cryopreservation agent*", or cryoprotectant, is a substance intended to protect the microorganism during freeze-thaw allowing it to maintain cell viability after freezing. Some common cryopreservation agents used for microorganism storage are: sucrose, glycerol, dimethyl sulfoxide (DMSO), among others.

"*Stock or storage medium*" refers to a fraction of microorganism in freezing medium stored for a determined period of time.

"*Supernatant*" is used hereafter to describe the liquid phase of a microorganism culture medium (growing or fermentation medium), free of particulate or solid material. The supernatant can be obtained by centrifuging and/or filtering the material, such process is well known on the state of the art.

"*Peptones*" are defined in the present invention as mixtures of compounds yielded by protein hydrolysis (it comprises proteins fragments, whose composition depends on the protein source used for hydrolysis). Commonly, peptones are obtained by acid or enzymatic hydrolysis of natural products, such as animal or vegetable tissues, as well as milk or microbial cultures.

The protein source for producing animal origin peptones is often a by-product of meat and dairy production. Therefore, as result of its hydrolysis, there are several other compounds, apart from peptides and amino acids, such as fats, metal ions, salts and vitamins.

*"Animal Product-Free"* is used to describe a substance completely absence of any animal origin product, component or compound. *"Animal"* includes mammals, birds, reptiles, fishes, amphibians, arthropods withal other animal species. *"Animal"* excludes microorganisms such as bacteria and yeasts. Thus, *"Animal Product-Free"* can comprise proteases derived from bacteria of the genus *Clostridium,* such as *C. histolyticum.* "*Animal Product-Free"* does not include animal-derived proteins, such as: immunoglobulin, meat digests and byproducts, and dairy products and their derivatives. On the context of this invention *"compound", "ingredient", "product"* or *"source"* "of non-animal origin" are the preferred sources of ingredients for the *Clostridium histolyticum* culture medium, including vegetables, microorganisms (e.g., yeast) and synthetic compounds.

*"Standard or traditional culture medium"* refers to the culture medium for growth and/or fermentation of bacteria of the genus *Clostridium,* in particular *C. histolyticum,* characterized for comprising animal-origin compounds, including peptone proteose, tryptic digests of casein or meat, casein peptone among others.

### Invention Description

It is known that in the case of *Clostridium histolyticum* growth, animal-origin peptones are considered essential ingredients in the culture medium, having a strong influence on the production of proteases with gelatinolytic and collagenolytic activity. However, regarding the protease production for therapeutic use in humans, it is ideal that animal-origin ingredients are avoided throughout the entire process, eliminating the risk of pathogens interspecies horizontal transmission and pathological induction of immune responses against animal-origin antigenic peptides that may be present in the final product, even after several purification steps.

Thereby, this invention describes an animal product-free *Clostridium* culture medium for growth and proteases production possessing collagenolytic and gelatinolytic activity. Most preferably, the present invention describes a culture medium for *C. histolyticum.*

Bacterial growth and protease production with collagenolytic and gelatinolytic activity using an animal product-free culture medium as described in this invention were compared to observations of the same culture parameter with culture medium comprising animal-origin ingredients such as proteose peptone or casein hydrolysate (known as standard or traditional culture medium).

Particularly, the culture medium described in the present invention yields a supernatant with higher collagenolytic and gelatinolytic activity than the one obtained from *Clostridium histolyticum* culture using standard culture medium (comprising animal-origin peptones).

Thereby, this invention presents a culture medium for bacteria of the genus *Clostridium,* most particularly C. *histolyticum,* animal product-free medium comprising water, non-animal origin peptone, or its derivatives, yeast extract, cysteine and arginine, or its pharmaceutically acceptable salts.

The culture medium of the present invention can additionally comprise one or more additive is selected from the group consisting of agent for adjusting pH within the range of 7.0 and 8.0, reducing agents, inorganic salts, vitamins and mixtures thereof.

Optimum levels of growth rate and proteases production presenting collagenolytic and gelatinolytic activity, mainly collagenase, are achieved using the culture medium described on this invention, having a pH ranging from 7.0 to 8.0. The culture medium pH is, preferably, around 7.0. pH adjustment must be performed after sterilization and before initiating *C. histolyticum* culture , through aqueous solutions of an agent for pH adjustment known by those skilled the art , e.g., ammonium sulphate, NaOH, sulphuric or nitric acid, among others.

The additives (reducing agents, inorganic salts and vitamins) that can be included on an animal product-free culture medium are selected among substances known by those skilled in the art. The reducing agent, according to this invention, is selected from the group consisting of sodium thioglycolate, sodium bisulphite, iron salts, glucose and mixtures thereof.

Additionally, according to this invention, the inorganic salts are selected from the group consisting of NaCl, KCl, Na₂HPO₄, NaH₂PO₄, KH₂PO₄, K₂HPO₄, MgSO₄, FeSO₄, MnSO₄ and mixtures thereof.

Vitamins, according to the present invention, is selected from the group consisting of biotin, pimelic acid, nicotinamide, calcium pantothenate, folic acid, thiamine nitrate, riboflavin, p-aminobenzoic acid (PABA) and mixtures thereof.

Non-animal origin peptones evaluated as ingredients for *Clostridium histolyticum* culture medium are listed in **Table 1.**

**Table 1. Non-animal origin peptones evaluated as ingredients for Clostridium histolyticum culture medium as described in the present invention**

| **Non-animal origin peptones** | **Supplier** |
|---|---|
| Amysoy® | Sheffield™ Bioscience (Kerry, USA) |
| HYP A® | BioSpringer (France) |
| Hy-Pep 1510® | Sheffield™ Bioscience (Kerry, USA) |
| Hy-Pep 1511® | Sheffield™ Bioscience (Kerry, USA) |
| Hy-Pep 4601® | Sheffield™ Bioscience (Kerry, USA) |
| Hy-Pep 4605® | Sheffield™ Bioscience (Kerry, USA) |
| Hy-Pep 5603® | Sheffield™ Bioscience (Kerry, USA) |
| Hy-Pep 7504® | Sheffield™ Bioscience (Kerry, USA) |
| Hy-soy® | Sheffield™ Bioscience (Kerry, USA) |
| NZ-Soy BL4® | Sheffield™ Bioscience (Kerry, USA) |
| NZ-Soy BL7® | Sheffield™ Bioscience (Kerry, USA) |
| Soybean peptone 312 - AF® | Biotecnica Internacional (Mexico) |

Non-animal origin peptones can also be obtained from other suppliers, such as: Soy peptone® (Gibco), Bac-soytone® (Difco), SE50M® (DMV), Peptona de soja 312® (Biotecnica International, Mexico), SE50MAF-UF® (DMV), Stedygro Soy® and Proyield Soy®.

The animal product-free culture medium for *Clostridium histolyticum* comprising a non-animal origin peptone that is a vegetable peptone selected from the group consisting of soybean, cotton, wheat, sunflower, rice, peanuts, beans, peas, potatoes, sweet corn or a mixture thereof. Preferably, the vegetable peptone is a soybean, cotton or wheat peptone, or its mixtures. Most preferably, the vegetable peptone is a soybean peptone.

This invention discloses the surprising observation that peptones, or other animal-origin components are not necessary in *Clostridium histolyticum* culture media, and that the culture medium of the present invention, animal product-free, yields proteases with collagenolytic and gelatinolytic activity superior to that obtained in conventional culture medium containing animal-origin components.

According to the present invention, vegetable peptones from different suppliers can be used in the animal product-free culture medium for *Clostridium histolyticum.* Preferably, the vegetable peptones are hydrolyzed and its production process is also animal product-free.

The vegetable peptone, according to this invention, is selected from the group consisting of Hy Soy®, Soytone®, Amisoy®, NZ Soy BL4®, NZ Soy BL7®, Hy Pep 1510®, Hy Pep 1511®, Hy Pep 5603®, Hy Pep 7504®, Stedygro Soy®, SE50M®, Proyield Soy®, SE50MAF-UF®, and mixtures thereof.

Preferably, the vegetable peptone is selected from the group consisting of Hy Pep 4601®, NZ soy BL4®, HyPep 1511® and Hy Pep 7504®. Most preferably, the vegetable peptone is NZ soy BL4®.

Besides non-animal or vegetable origin peptones, the culture medium developed and described in the present invention for *Clostridium histolyticum* culture contains yeast extract as critical ingredient. Commercial suppliers evaluated for this ingredient are listed in **Table 2.**

**Table 2. Yeast extracts evaluated as ingredients for Clostridium histolyticum culture medium described on this invention.**

| **Yeast Extract** | **Supplier** |
|---|---|
| Bacto YE® | BD Bioscience (USA) |
| Hy-Pep YE® | Sheffield™ Bioscience (Kerry, USA) |
| Hy-Yeast 412® | Sheffield™ Bioscience (Kerry, USA) |
| Hy-Yeast 413® | Sheffield™ Bioscience (Kerry, USA) |
| Hy-Yeast 502® | Sheffield™ Bioscience (Kerry, USA) |
| Hy-Yeast 501® | Sheffield™ Bioscience (Kerry, USA) |
| Hy-Yeast 503® | Sheffield™ Bioscience (Kerry, USA) |
| Prodex 710 SD® | BioSpringer (France) |
| Prodex NS70SD® | BioSpringer (France) |
| Pronal 5001® | BioSpringer (France) |
| YE 11® | Sheffield™ Bioscience (Kerry, USA) |
| YE151® | Biotecnica Internacional (Mexico) |
| YE251® | BioSpringer (France) |
| YE 70161® | Fluka (Sigma-Aldrich GmbH) |
| YE Y42S0® | Sigma-Aldrich GmbH |
| Synth® | Synth® (Brazil) |
| YE 207® | BioSpringer (France) |
| YE 5114® | Vetec Quimica Fina (Brazil) |

Preferably, according to the present invention, the yeast extract is selected from the group consisting of Bacto YE®, Hy-Pep YE®, Hy-Yeast 412®, Hy-Yeast 413®, Hy-Yeast 502®, Hy-Yeast 501®, Hy-Yeast 503® Prodex 710 SD®, Prodex NS70SD®, Pronal 5001®, YE 11®, YE 151®, YE 251®, YE 70161®, YE Y4250®, Synth®, YE 207®, YE 5114® and mixtures thereof. Most preferably, the yeast extract is YE 251®.

Regarding the amino acids, arginine is L-arginine and cysteine is L-cysteine or its pharmaceutically acceptable salts. Preferably, the pharmaceutically acceptable salts are cysteine hydrochloride and arginine hydrochloride.

The culture medium of the present invention, quantitatively, is characterized by comprising non-animal origin peptone at a concentration of 0.5% to 5% w/v; yeast extract present at a concentration of 0.5% to 5% w/v; cysteine, or its pharmaceutically acceptable salts, at a concentration of 0.01% to 0.1% w/v; and arginine, or its pharmaceutically acceptable salts, at a concentration of 0.1% to 1% w/v.

Preferably, the culture medium comprising non-animal origin peptone at a concentration between 1% and 4% w/v, yeast extract at a concentration between 1% and 4% w/v, cysteine, or its pharmaceutically acceptable salts, at a concentration of 0.03% to 0.07% w/v, and arginine, or its pharmaceutically acceptable salts, at a concentration ranging from 0.25% to 0.7% w/v. Most preferably, the culture medium comprising non-animal origin peptone at a concentration of 3% w/v, yeast extract at a concentration of 3% w/v, cysteine, or its pharmaceutically acceptable salts, at a concentration of 0.0625% w/v, and arginine, or its pharmaceutically acceptable salts, at concentration of 0.375% w/v.

The culture medium, according to the present invention, must be sterilized before the inoculum with *C. histolyticum.* Media sterilization can be performed through methods known by those skilled in the art, for example, treatment by moist heat autoclaving or filtration. Thus, the culture medium described in this invention is characterized by being sterile. Preferably, the animal product-free culture medium of the present invention is characterized by being liquid.

The culture medium, according to the present invention, is used on a process for producing a supernatant from a *Clostridium histolyticum* liquid culture comprising one or more protease presenting collagenolytic and gelatinolytic activity.

*C. histolyticum,* when cultured in the animal product-free liquid medium presented herein, secrete such proteases into the liquid phase. Collagenases, along with other proteases, are the main protein components in the supernatant produced by such *C. histolyticum* culture.

On another aspect of the present invention, a process for producing supernatant of *Clostridium histolyticum* liquid culture is described, comprising one or more proteases with collagenolytic and gelatinolytic activity and which comprising the *C. histolyticum* culture in an animal product-free culture medium according to the present invention.

The process for producing supernatant of *Clostridium histolyticum* liquid culture comprising one or more proteases with collagenolytic and gelatinolytic activity comprising the following steps:
**a)** providing a sterile animal product-free culture medium;
**b)** culturing an aliquot of Clostridium histolyticum stock culture in the culture medium in step (a), under anaerobic conditions at about 37°C before reaching the stationary growth phase in order to obtain an inoculum;
**c)** providing a sterile animal product-free culture medium for *Clostridium* bacteria, according to the present invention
**d)** adding to the culture medium in step (c) a volume of inoculum from step (b) equal to or lower than 10% of the final volume of the medium defined in step (c);
**e)** culturing the *C. histolyticum* obtained in step (d) under anaerobic conditions at about 37°C until the stationary growth phase is attained;
**f)** removing cellular material and other particulate matter in the liquid phase in step (e) yielding a supernatant comprising one or more proteases with collagenolytic and gelatinolytic activity.

Anaerobic conditions, as required in steps (b) and (e) can be attained through various methods, including but not limited to the ones described hereafter. Methods for maintaining anaerobic conditions for microorganism growth are well known by those skilled in the art. For instance, the anaerobic conditions in steps (b) and (c), can be maintained through the addition of nitrogen gas to the culture medium during cell growth.

The purpose of culture medium referred in step (a) is to increase cell count from a stock culture, being stored under appropriate conditions for maintaining cell viability. Preferably, the stock culture is maintained at -80°C in an animal product-free culture medium containing one or more cryopreservation agents. In order to achieve *C. histolyticum* best growth rates in an animal product-free medium, it is ideal that the stock medium is also composed of non-animal derived compounds.

The stored stock culture is produced from an animal product-free culture medium comprising vegetable peptones, preferably soybean peptone or its derivatives, yeast extract, cysteine, as well as a cryopreservation agent, in pH between 6.5 and 8.0 and. Cryopreservation agents for microorganisms, mainly bacteria, are broadly known by person skilled in the art and can be selected from the group consisting of glycerol, sucrose, dimethyl sulfoxide and glucose. The amount of cryoprotectant added to the stock culture varies depending on the bacterial lineage and must be correctly adjusted prior to preparing the stock microorganism bank (master cell bank and working cell bank).

The purpose of the growth phase (step (b)) is to increase the amount of available and viable microorganisms for production proteases with collagenolytic and gelatinolytic activity, which occurs in step (e). Additionally, the growth phase allows the dormant microorganisms in the stock culture to grow actively and secrete proteases with collagenolytic and gelatinolytic activity at step (e). The sterile and animal product-free culture medium of step (a) of the process described above is characterized by comprising water, vegetable-origin peptone, yeast extract and cysteine. Specifically, the sterile and animal product-free culture medium of step (a) comprising water, 0.5 to 5% w/v of vegetable peptone, 0.5 to 5% w/v of yeast extract, 0.01 to 0.1% w/v of cysteine or its pharmaceutically acceptable salts and pH of 6.5 to 8.0. Preferably, the culture medium of step (a) is characterized by comprising water, 3% w/v of vegetable peptone, 3% w/v of yeast extract, 0.0625% w/v of cysteine or its pharmaceutically acceptable salts and pH of 6.5 to 8.0.

As it will be noticed in the Examples 1 and 2 described below, there is no need of adding arginine to the animal product-free culture medium in order to obtain a higher growth rate than the one observed in standard culture medium (with animal-origin peptone). Therefore, the preferred culture medium described for step (a) has been defined considering only the minimum composition in order to obtain the presented results, in other words, only the components considered essential for growth. However, similar results are observed when the medium contains, not only the described essential compounds, but also arginine and other additives (inorganic salts, vitamins and reducing agents).

The arginine amino acid is essential for the surprising results of the present invention regarding the production of proteases with collagenolytic and gelatinolytic activity (step (e)). It has been observed that the production of proteases with collagenolytic and gelatinolytic activity obtained in a *C. histolyticum* culture supernatant is much higher when the culture medium contains arginine and cysteine when compared with the non-animal origin medium (only with cysteine) and with the traditional medium containing animal origin compounds.

Thus, the process for producing supernatant of *Clostridium histolyticum* liquid culture comprising one or more proteases with collagenolytic and gelatinolytic activity is characterized by the fact that the sterile animal product-free culture medium described in step (c) comprising water, vegetable peptone, yeast extract, cysteine and arginine, with concentrations defined by the present invention, and a pH of 7.0 to 8.0.

Preferably, the process for producing supernatant of *Clostridium histolyticum* liquid culture comprising one or more proteases with collagenolytic and gelatinolytic activity is characterized by the fact that the sterile animal product-free culture medium described in step (c) comprising water, 3% w/v of vegetable peptone, 3% w/v of yeast extract, 0.0625% w/v of cysteine or its pharmaceutically acceptable salts, 0.375% w/v of arginine or its pharmaceutically acceptable salts and a pH between 7.0 and 8.0.

Bacterial growth can be monitored by measuring the optical density of the culture medium during the process described above (steps (b) and (e)) using a spectrophotometer (reading at 600 nm) according to methods well known by those skilled in the art.

The *C. histolyticum* culture during step (b) may occur in one or more steps. Preferably, the process for producing supernatant of *Clostridium histolyticum* liquid culture is characterized by the fact that the step (b) comprising at least the following steps: 1) cultivating the stock culture in a medium culture from step (a) in a ratio of 0.1:1.0 v/v for about 16 hours in order to obtain a pre-inoculum; 2) adding to the pre-inoculum a volume of the culture medium that is equal or superior to the double of the culture medium volume from step (1) and maintaining the culture for about 12 hours in order to obtain an inoculum.

It is essential that the growth during step (b) does not lead to cell lysis before its inoculation into the culture medium in step (c), guaranteeing the cell viability of the inoculum.

The process for producing supernatant of *Clostridium histolyticum* liquid culture can be characterized by comprising only steps from (c) to (f), initiating the *C. histolyticum* culture directly from a stock culture defined in step (c), without the need of producing an inoculum.

Preferably, the process for producing supernatant of *Clostridium histolyticum* liquid culture is characterized by the fact that the *C. histolyticum* culture during step (e) involves the addition, in sufficient amount, of one or more known agents which allow pH adjustment to maintain the pH between 6.5 and 8.0.

In the present invention, during step (f) of the process for obtaining *C. histolyticum* collagenolytic enzymes, the separation of cellular material or other particulate matter from the culture medium liquid phase can be performed through different techniques of filtration, centrifugation or chromatographic purification known on the state of the art, not limited to tangential filtration, centrifugation, chromatography gel filtration, among others.

Thus, the process for producing supernatant of *Clostridium histolyticum* liquid culture is characterized by the fact that step (f) is performed through filtration or centrifugation procedures, or both.

In addition, the process for producing supernatant of *Clostridium histolyticum* liquid culture is characterized by comprising one or more additional steps for the purification of one or more proteases with collagenolytic and gelatinolytic activity in the supernatant obtained in step (f). Purification can be performed through various filtration methods, chromatographic purification or saline precipitation, not limited to ammonium sulfate precipitation, tangential filtration, ion-exchange chromatography, affinity chromatography, gel filtration chromatography, among others, individually or combined.

According to the present invention, the supernatant of the *C. histolyticum* liquid culture is a complex mixture including, not only proteases with collagenolytic and gelatinolytic activity, mainly collagenases, but also other proteins, bacterial metabolites, culture medium components, among other substances.

Thus, an additional aspect of this invention refers to the supernatant of *Clostridium histolyticum* liquid culture comprising one or more proteases with collagenolytic and gelatinolytic activity characterized by being produced through a process described above.

Particularly, the supernatant, according to the present invention, is characterized by comprising 20 to 90% of collagenases after the purification.

The supernatant, according to this invention, can be directly purified in order to obtain one or more proteases with collagenolytic and gelatinolytic activity. Alternatively, the supernatant can be stored on a frozen or lyophilized form, without, losing its collagenolytic and gelatinolytic activity.

In another aspect, the invention discloses the use of a supernatant of *Clostridium histolyticum* liquid culture obtained through the process described in the present invention characterized by being for the preparation of a medicament for the treatment of diseases that benefit from collagenolytic and gelatinolytic activity of proteases of *C. histolyticum.* Particularly, the invention describes the use of a supernatant of *C. histolyticum* liquid culture characterized by being for the preparation of a medicament for the treatment of collagen-mediated diseases or diseases due to accumulation of fibrous tissue. In addition, the invention describes the use of a supernatant of the *C. histolyticum* liquid culture characterized by being for the preparation of a medicament for the treatment of necrotic tissue and/or healing processes. Besides the use of a supernatant of the *C. histolyticum* liquid culture is characterized by being in the preparation of a medicament for the enzymatic debridement.

Burns and skin lesions of diverse nature are examples of situations where necrotic tissue treatment is necessary.

This invention also discloses a pharmaceutical composition characterized by comprising as active ingredient the supernatant of *Clostridium histolyticum* liquid culture obtained from the process described herein and pharmaceutically acceptable excipients. The pharmaceutical composition can include one or more excipients pharmaceutically acceptable, caps, plugs, carriers, stabilizers, preservatives and thickeners. The pharmaceutical composition, according to the invention, is characterized by being for administration in animal or human, for therapeutic or cosmetic use.

According to this invention, the pharmaceutical compositions comprising as active ingredient the supernatant of *Clostridium histolyticum* liquid culture can be administered through oral, sublingual, nasal, intravenous, intramuscular, intraperitoneal, intrarticular, subcutaneous, dermal, transdermal, among others. Preferably, the pharmaceutical composition, according to the present invention, is characterized for being formulated for subcutaneous, dermal and transdermal administration.

Most preferably, the pharmaceutical composition, according to the present invention, is characterized by being for topical use and comprising an activity potency ranging from 0.2 U to 1.8 U of collagenase per gram of composition (quantified through the colorimetric method with ninhydrin).

The carriers or pharmaceutically acceptable excipients are selected based on the final composition that may be presented as capsules, tablets, oral solutions, solutions for nasal administration, injectable solution for intramuscular, intravenous, cutaneous or subcutaneous or yet solutions, ointments or creams for topical use, among others. Thus, the pharmaceutical compositions of this invention comprises as active ingredient the supernatant of *Clostridium histolyticum* liquid culture, pharmaceutically acceptable excipients or carriers in the form of a cream or ointment formulation, lyophilized or aqueous solutions. Excipients, carriers or stabilizers pharmaceutically acceptable do not demonstrate toxicity to the recipient organism at the dosages and concentrations used and can include buffers such as phosphate, citrate, and other organic acids; antioxidants such as ascorbic acid and methionine; preservatives such as octadecyldimethylbenzyl ammonium chloride, hexamethonium chloride, benzalkonium chloride, benzethonium chloride, phenol, butyl alcohol, benzyl alcohol, alkyl parabens such as methyl- and propylparaben, catechol, resorcinol, cyclohexanol, 3-pentanol, and m-cresol; amino acids, monosaccharides, disaccharides, and other carbohydrates such as glucose, mannose, sucrose, mannitol, or sorbitol; polysaccharides (such as the polysaccharide hydrophilic Ficoll®), polymeric excipients such as polyvinylpyrrolidones, dextrins, polyethylene glycols, flavoring agents, sweeteners, anti-static agents, chelating agents such as EDTA or EGTA; salts releasing ions like sodium, metal complexes, non-ionic polysorbates such as "TWEEN 20" and "TWEEN 80", lipids such as phospholipids, fatty acids and steroids such as cholesterol. Methods for the preparation of pharmaceutical compositions are well known, or will be apparent in light of this invention, to those skilled in the art in pharmaceutical technology.

Such pharmaceutical compositions find application in the treatment of diseases that benefit from collagenolytic and gelatinolytic activity of *C. histolyticum* proteases, in the treatment of pathological conditions of the human body associated with excessive collagen deposition and erratic accumulation of fibrous tissue rich in collagen, treatment of necrotic tissue and healing process and treatment for enzymatic debridement.

*C. histolyticum* culture supernatant, as well as proteases with collagenolytic and gelatinolytic activity, mainly collagenases, produced according to the process described in this invention does not exhibit toxicity, fact proven by cytotoxicity and dermal toxicity studies.

For a better understanding of the teachings of this invention, examples 1 to 8 are presented without, however, limiting its scope.

**Examples 1-4** demonstrate the preparation of an animal product-free culture media, according to the present invention, and the *C. histolyticum* growth and proteases with collagenolytic and gelatinolytic activity production. Results obtained confirm the effectiveness of animal product-free culture medium, related to *C. histolyticum* growth in comparison with standard culture medium, even without arginine in its composition(Figures 1 and 2). The importance of the yeast extract in the medium's composition can be observed in Figure 3, and the preferred pH range for *C. histolyticum* growth is exemplified in Figure 4. Figures 5 and 6 clearly demonstrate that supplementing the animal product-free medium with vitamin and inorganic salts yields no improvement in cell growth nor in protein expression. For such reason such components are considered optional. However, the sole addition of arginine in the animal product-free culture medium proposed herein is critical for increasing protease expression (yielding a supernatant with higher collagenolytic and gelatinolytic activities) this is demonstrated in Figures 7 and 8. Evidence of the importance of arginine and cysteine for producing proteases with collagenolytic and gelatinolytic activity in the animal product-free culture medium is shown in Figure 9.

Moreover, the examples cited above clearly portray the quality of the supernatant obtained by culturing *C. histolyticum* in animal product-free medium. This quality is related to the amount of proteases with high molecular weight, which is associated with the *C. histolyticum* collagenases, whose enzymatic activity is also demonstrated.

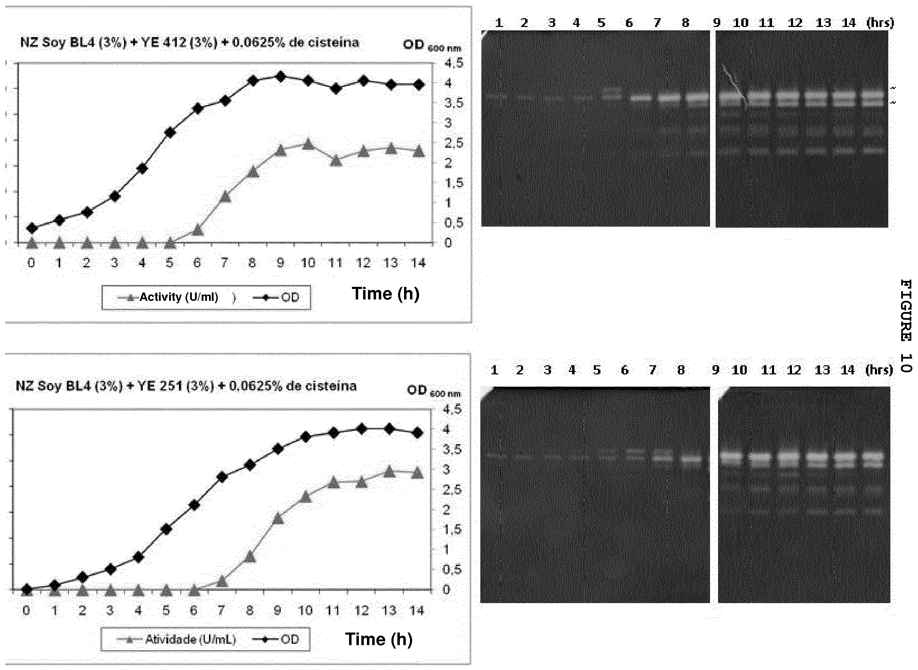

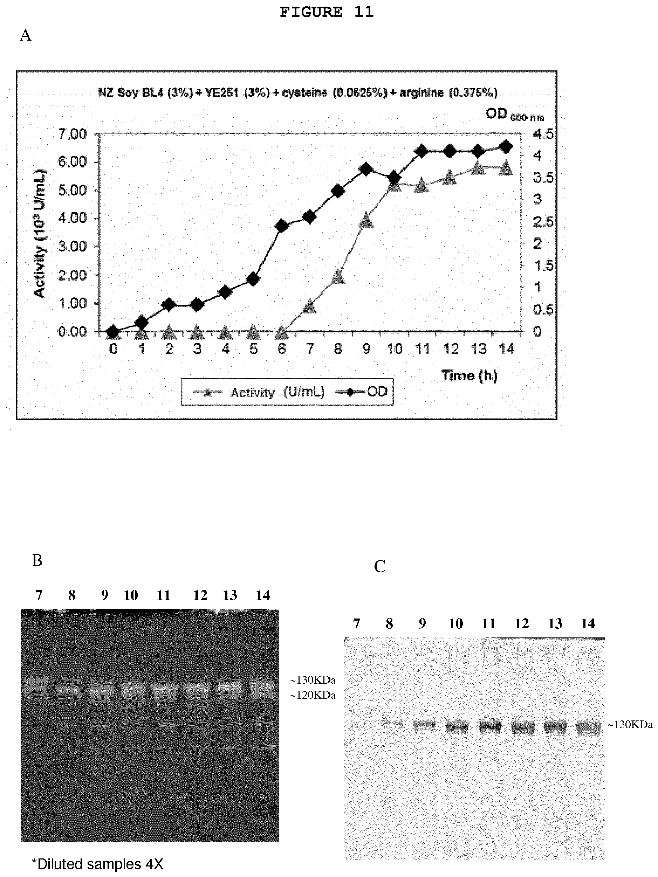

**Example 5** shows the results of culturing in bioreactor (3L) using the animal product-free culture medium described in the present invention. Figure 10 shows the results of a cell culture using animal product-free culture medium without arginine, demonstrating the smaller protease production when compared to one in which arginine was added (Figure 11) . Figure 12 shows a comparative graph between animal product-free culture media with and without arginine. The parameters considered in this analysis were bacterial growth rate and enzyme production.

**Example 6** describes a recovery process to the proteases with collagenolytic and gelatinolytic activity present in the supernatant from the *C. histolyticum* culture in animal product-free medium described in the present invention. The quality of the supernatant obtained can be highlighted, with no precipitation, facilitating its manipulation. The results of the collagenolytic enzymes recovery by precipitation with ammonium sulfate, followed by dialysis and lyophilization are shown (Figure 13).

**Example 7** presents a pharmaceutical composition for topic use comprising the supernatant obtained from *C. histolyticum* liquid culture produced in animal product-free culturemedium. The stability of the compositions is also described; substantiating the quality of the product obtained utilizing the described fermentative processes and defining one more advantageous trait of the present invention.

**Example 8** describes assays of dermal toxicity with repeated doses (21 days) in rats (male and female), demonstrating the absence of toxicity of the pharmaceutical compositions prepared with collagenolytic enzymes obtained through the lyophilization of the supernatant obtained from *C. histolyticum* liquid culture produced in animal product-free culture medium, according to the present invention, and through tangential filtration. The absence of pharmaceutical compositions toxicity highlights, once again, the quality of the enzymatic composition of the supernatant from cultures of *C. histolyticum* in animal product-free culture media, according to the present invention (Figures 14-17).

**Example 9** shows the results of the cultivation of two *C. histolyticum* strains, ATCC21000 and T248 in a 3L bioreactor using animal product-free medium as described in the present invention. Figure 14 shows the comparative results, demonstrating an equivalent production of colagenolytic and gelatinolytic proteases by both strains.

**Example 10** shows the cytotoxicity test results of the colagenolytic and gelatinolytic proteases obtained from the supernatant of the two *C. histolyticum* strains liquid culture (ATCC 21000 and T248) grown in animal product-free culture medium.

### EXAMPLES

The examples below are merely illustrative, and must be applied solely for a better understanding of the constant developments in the present invention, and are not to be used with the intention of limiting the described objects.

*C. histolyticum* strains may be obtained from several sources, including the American Type of Culture Collections (ATCC). Various *Clostridium histolyticum* strains have already been applied with the intent of obtaining collagenase; hence the present invention does not limit itself only to the strain utilized for the demonstration through the examples below.

The ATCC 21000 (non-mobile mutant of a mobile lineage, wild, isolated from the soil ATCC 21000™) *Clostridium histolyticum* strain was used in all examples presented herein. The reason for such is due to the fact that this strain is known for yielding high amounts of good quality collagenolytic and gelatinolytic enzymes. The culture medium advised by ATCC for its propagation includes animal origin peptones, inorganic salts and leaver digest (culture medium Reinforced Clostridial Medium - RCM - Oxoid CM149, pH 8.0; anaerobic conditions).

In addition, Examples 9 and 10 show the results of cultures using animal product-free culture medium as described herein for the T248 *Clostridium histolyticum* strain, isolated from topsoil sample of a private area (Espirito Santo do Pinhal/SP, Brazil). The T248 strain was characterized morphologically (gram positive bacillus, sporulated), biochemically (optional anaerobic, which degrades gelatins) and genetically (*tpi* gene and 16S, 23S ribosomal and 16S and 23S intergenic regions), present exclusively in *C. histolyticum* strains encoding collagenase class I and II. The strain was also characterized phylogenetically using 16S-23S intergenic region sequences.

### Example 1. Process for producing standard and animal product-free culture media for Clostridium histolyticum.

The standard or traditional culture medium was prepared using the following ingredients, quantities refer per liter of medium: Proteose peptone n. 3 or casein peptone (20 g); yeast extract (5 g); glucose (5 g); sodium thioglycolate (250 mg), pH 7.0 using 5M NaOH.

The animal product-free culture medium was prepared using the following ingredients per liter: vegetable origin peptone (10-40 g); yeast extract (0-30 g); cysteine hydrochloride (625 mg), pH 7.0 using 5M NaOH. All components, except glucose which is present only in the standard medium, were dissolved in water and the pH was adjusted to 7.0 using 5M NaOH. The medium was then sterilized in autoclave at 121°C for 20 minutes. A glucose solution was prepared separately and sterilized in an autoclave (autoclave at 121oC for 20 minutes) and this solution was then added to the standard medium only at the time of culture.

Several amounts of different vegetable origin peptones and yeast extract were evaluated as compounds in an animal product-free culture medium described above.

Moreover, the influence of different pH's in the animal product-free culture medium (3% w/v NZ soy BL4, 3% w/v YE251 e 0.0625% w/v cysteine hydrochloride, pH 7.0) was also investigated, being the evaluated pH range from 6.5 to 8.5.

### Example 2. C. histolyticum growth in an animal product-free culture medium and in a standard culture medium as defined in Example 1.

Initially, the frozen stock culture of *Clostridium histolyticum* (100 pL) was suspended in 1 mL of standard culture medium or 1 mL of animal product-free culture medium (described in Example 1), separately in two tubes that were filled with the respective culture media to reach the 10 mL final volume (culture denominated as inoculum). Tubes containing the inoculum were incubated at 37 ºC, in anaerobiosis, for approximately 16 hours.

After this period, 3 mL of the inoculum were incubated in 250 mL vials, containing 150 mL of the respective *C. histolyticum* culture media. The culture was then maintained at 37 ºC for 12-14 hours in under anaerobic conditions (N₂ addition). All the analyses presented were carried out at the end of the 12-14 hour interval. Bacterial growth was determined through analysis of optical density - OD - using a spectrophotometer with a set wavelength of 600 nm.

Figure 1 shows the OD (optical density) results for *C. histolyticum* (ATCC 21000) growth in an animal product-freeculture medium comprising vegetable peptone (3% w/v) and yeast extract (3% w/v) from different suppliers. The evaluated suppliers display similar results of bacterial growth. In Figure 1 it is clearly observed a significant increase in the OD measured when animal product-free culture medium was used, indicating higher bacterial growth rate when compared to the standard medium (with animal origin peptones) (Vetec + Casein). All animal product-free culture media represented in Figure 1 contain 625 mg/L of cysteine hydrochloride and pH of approximately 7.0.

The results for growth rates analysis when *C. histolyticum* is subjected to animal product-free culture medium considering various vegetable peptones (Hy-pep 1511; 1-3% w/v) and yeast extract (YE 251; 0-3% w/v) concentrations is presented in Figure 2. All animal product-free culture media represented in Figure 2 contain 625 mg/L of cysteine hydrochloride and pH of approximately 7.0. Some variation in the *C. histolyticum* growth rate is observed in among these animal product-free culture media. However, the OD values reached are always superior to those obtained when culturing *C. histolyticum* in a standard medium (with animal origin peptone).

The importance of the yeast extract in the composition of the animal product-free culture medium is demonstrated in Figure 3 (animal product-free culture media comprising 0, 1, 2 or 3% of yeast extract). It can be observed that the absence of yeast extract in the culture medium's composition inhibits the bacterial growth as well as protease production with collagenolytic and gelatinolytic activity, even by increasing vegetable peptone amount in its composition (evaluated concentrations of vegetable origin peptone: 3 and 4%). All animal product-free culture media represented in Figure 3 contain 625 mg/L of cysteine hydrochloride and pH of approximately 7.0. The evaluation of the collagenolytic and gelatinolytic activity was carried out in accordance to what is described in Example 4.

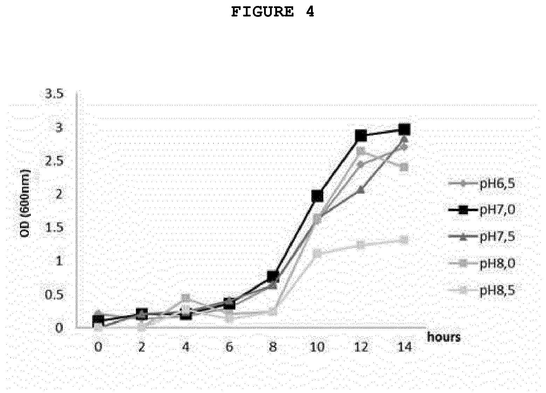

Figure 4 represents the results of bacterial growth obtained through the culturing of *C. histolyticum* in growth medium with pH ranging from 6.5 to 8.5. In the pH range evaluated, only the most basic pH (pH 8.5) negatively influenced the bacterial growth.

### Example 3. Production of animal product-free culture medium for Clostridium histolyticum and standard culture medium for production of a supernatant comprising proteases with collagenolytic and gelatinolytic activity.

The culture medium was prepared according to Example 1.

The animal product-free culture medium of the present invention was prepared using the following ingredients per liter: vegetable origin peptone (30 g); yeast extract (30 g); cysteine hydrochloride (0.625 g); arginine hydrochloride (3.75 g) and pH 7.0 (adjusted with 5M NaOH).

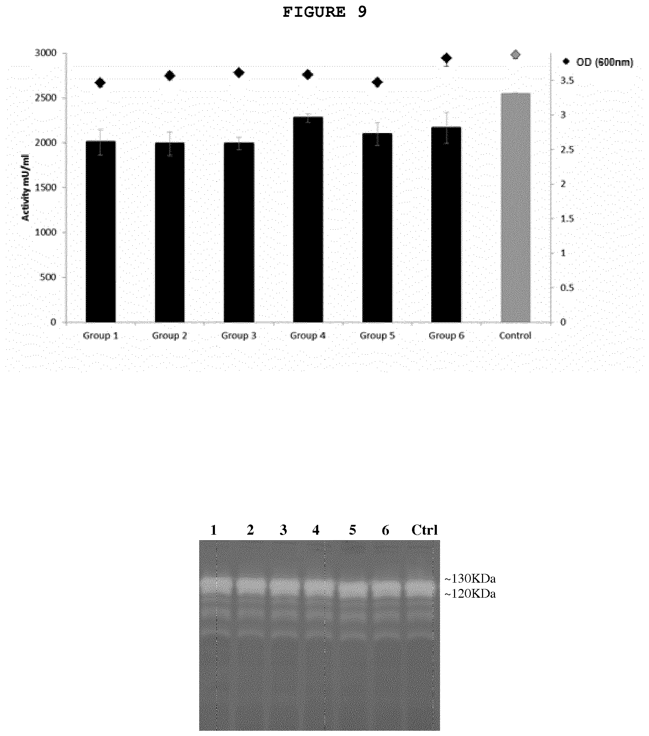

Experiments to demonstrate the surprising effect of the animal product-free culture medium in *C. histolyticum* growth defined by present invention and described above were carried out in five different types of culture media comprising the following components per liter:
- Medium 1: soybean peptone (30 g; NZ-Soy BL4®); yeast extract (30 g; YE 251®); cysteine hydrochloride (0.625 g), pH 7.0 (adjusted with 5M NaOH).
- Medium 2: soybean peptone (30 g; NZ-Soy BL4®); yeast extract (30 g; YE 251®); cysteine hydrochloride (0.625 g), pH 7.0 (adjusted with 5M NaOH) and additional salts and/or amino acids (described in Figure 5).
- Medium 3: soybean peptone (30 g; NZ-Soy BL4®); yeast extract (30 g; YE 251®); cysteine hydrochloride (625 mg), pH 7.0 (adjusted with 5M NaOH) and additional amino acids (Groups 1 to 6, described in Figure 9).
- Medium 4: soybean peptone (30 g; NZ-Soy BL4®); yeast extract (30 g; YE 251®); cysteine hydrochloride (0.625 g), pH 7.0 (adjusted with 5M NaOH) and additionally glycine and/or arginine.
- Medium 5: soybean peptone (30 g; NZ-Soy BL4®); yeast extract (30 g; YE 251®); cysteine hydrochloride (0.625 g), pH 7.0 (adjusted with 5M NaOH) and additional inorganic salts and vitamins (described in Figure 6).

Other vegetable origin peptones were evaluated relative to the culturing of *C. histolyticum* for the production of proteases with collagenolytic and gelatinolytic activity, showing results similar to NZ-Soy BL4®. In the same way, the different yeast extracts evaluated showed similar results to those of the YE 251® used in the animal product-free culture media.

The vegetable peptones and the yeast extracts suitable to provide the animal product-free culture medium are not limited to the ones used in the examples of this invention. Vegetable peptones and yeast extracts that demonstrate good solubility in water can be used in the preparation of the culture medium for *C. histolyticum* according to the present invention, since they are animal product-free.

After solubilizing all components of the animal product-free culture medium as described above (except glucose, present only in the standard medium) in water and adjustment of the pH to 7.0 (5M NaOH), the culture medium was sterilized in autoclave at 121 °C for 20 minutes.

### Example 4. Production of supernatant of Clostridium histolyticum liquid culture comprising one or more proteases with collagenolytic and gelatinolytic activities in an animal product-free culture medium defined in Example 3 and standard culture medium defined in Example 1.

*C. histolyticum* culture in an animal product-free culture medium was carried out as described in Example 2. The culture media evaluated are described in Example 3 (media 1 to 5).

After 12-14 hours of *C. histolyticum* culture in the animal product-free culture media described in Example 3, the bacteria cells were centrifuged and the collagenolytic and gelatinolytic activity of the proteases contained in the supernatant was quantified. Eight milliliters (8 mL) of the bacterial cultures were centrifuged at 500 rpm for 30 minutes at 4 °C for the removal of the insoluble material (cells, cell debris, precipitate, among others).

The protein profile in the supernatant was evaluated through electrophoresis in 7.5% SDS PAGE under non-reducing conditions (dyed with silver nitrate). The enzymatic activity profile (non-specific) was evaluated in non-reductive SDS-PAGE comprising 10% of gelatin and dyed with Coomassie blue (zymography). Both analytical methodologies are broadly known by those skilled in the art.

The collagenolytic and gelatinolytic activity of the obtained supernatants was quantified through EnzChek® Gelatinase/Collagenase Assay Kit (Molecular Probes, Invitrogen). Assays were carried out in microplates according to manufacturer instructions, using gelatin and/or collagen fluoresceine conjugated as substrate. Fluorescence determinations were made using a fluorescence reader. Briefly, 100 µL of the culture supernatant, after centrifugation, were mixed to the substrate diluted in the incubation buffer prepared according to the manufacturer instructions. Samples were incubated at 37 ºC for 15 minutes and the fluorescence was determined at 515 nm using a fluorescence microplate reader. As a negative control sterile culture medium was used instead of the culture supernatant. As a positive control, it was used a Collagenase reagent provided with the kit and prepared according to manufacturer instructions.

Bacterial growth was determined by optical density - OD - using a spectrophotometer at 600 nm.

Initially, the animal product-free culture medium supplemented (medium 2; comprising vegetable peptone, yeast extract and cysteine) with vitamins, inorganic salts and amino acids was evaluated. This experiment demonstrated a small influence of these additives in cell growth as well as protease expression with collagenolytic and gelatinolytic activities from supernatant of *Clostridium histolyticum* liquid culture (Figure 5). Medium 1 was used as a control for this assay.

The addition of vitamins, inorganic salts and amino acids to the animal product-free culture medium described in this invention (medium 5; comprising vegetable peptone, cysteine and arginine) also resulted in a small variation of the biomass (OD) and collagenolytic and gelatinolytic activity of this culture supernatant. Figure 6 shows these results, confirming that such vitamin and salt supplementation is optional to the present invention's culture medium. Medium 1 was used as a control of this assay.

Qualitative variations (different suppliers) of the vegetable peptone and yeast extract components were also evaluated (Medium 1 comprising vegetable peptone, yeast extract and cysteine) regarding the supernatant production of a *C. histolyticum* culture comprising proteases with collagenolytic and gelatinolytic activities. Figure 7 shows that the results of collagenolytic and gelatinolytic activities obtained for these cultures are of the same magnitude as the results obtained for the *C. histolyticum* culture in standard medium (with animal peptone). Thus, it is observed the need of an additional ingredient to the culture medium in order to obtain increased collagenolytic and gelatinolytic activity observed in the supernatant of the *C. histolyticum* liquid culture, which has been solved by the present invention's culture medium.

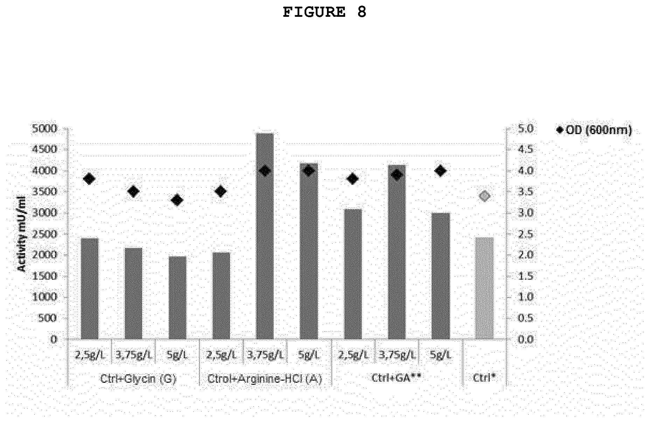

Figure 8 demonstrates that the *C. histolyticum* culture in an animal product-free culture medium as described in the present invention, comprising amino acids cysteine and arginine (medium 4), results surprisingly in a supernatant with significantly higher collagenolytic and gelatinolytic activity than the supernatant obtained when using animal product-free culture medium containing only the amino acid cysteine (Medium 1, used as a control of this assay). Such effect is due to arginine since the addition of another amino acid, for instance glycine, leads to reduction of collagenolytic and gelatinolytic activity observed.

Figure 9 shows results of collagenolytic and gelatinolytic activity and OD (600 nm) observed in *C. histolyticum* culture in medium 3 (animal product-free culture medium comprising cysteine and amino acid groups). As a control, a culture using medium 1 was carried out (animal product-free culture medium containing only the amino acid cysteine). It can be observed that no amino acid or amino acid combination added to the culture medium was able to increase collagenolytic and gelatinolytic activity, which was surprisingly observed in the supernatant from *C. histolyticum* liquid culture comprising amino acid arginine (Figure 8).

Additionally, it can observed that just 14 hours of fermentation are sufficient to obtain the supernatant comprising collagenolytic and gelatinolytic enzymes in suitable levels for industrial production, a period inferior to the one generally used, being this a very important parameter for process of production in industrial scale.

### Example 5. Production of a Clostridium histolyticum liquid culture comprising proteases with collagenolytic and gelatinolytic activities in an animal product-free culture medium - bioreactors culture.

Initially, the frozen stock culture of *Clostridium histolyticum* (100 pL) was suspended in 1 mL animal product-free culture medium (medium 1, as defined below), and then the culture medium was added to final volume of 10 mL (culture denominated as pre-inoculum). Tubes containing the pre-inoculums were incubated at 37ºC under anaerobic conditions for approximately 16 hours.After this period, 3 mL of the aforesaid pre-inoculum was then incubated in 250 mL flasks, containing 150 mL of the respective *C. histolyticum* culture medium. This was denominated inoculum, and it was maintained at 37 ºC during 12-14 hours under anaerobic conditions (attained through the addition of nitrogen gas).

For the cultures which were carried out in bioreactor (New Brunswick), 60 mL of the inoculum were transferred to 3 L of *C. histolyticum* culture medium. As before, such cultures were incubated at 37 ºC under anaerobic conditions, through the addition of nitrogen gas. The pH was monitored and controlled during the entire fermentation period being maintained in a range of 6.5 and 8.0. Such maintenance was achieved through the addition of 5M NH₄OH or 2M H₂SO₄. The culture was maintained for about 14 hours, period in which culture has reached its stationary growth phase for at least 2 hours.

Two culture media were evaluated, and their composition per liter is as described below:
- Medium 1: soy peptone (30 g; NZ-Soy BL4®); yeast extract (30 g YE 251®); cysteine hydrochloride (0.625 g) and pH 7.0 (adjusted with 5 M NH₄OH after sterilization).
- Medium 6: soy peptone (30 g; NZ-Soy BL4®); yeast extract (30 g YE 251®); cysteine hydrochloride (0.625 g), arginine hydrochloride (3.75 g) and pH 7.0 (adjusted with 5 M NH₄OH after sterilization).

The process for obtaining a *C. histolyticum* culture supernatant with collagenolytic and gelatinolytic activities according to the present invention is detailed below.
1 - Cell bank preparation: a cell bank was prepared using ATCC 21000 strain. It was grown in agar plates in which the medium was composed of: soy peptone, yeast extract and agar. Colonies with apparent higher cell growth were isolated and inoculated in cell bank medium containing the same ingredients as the solid medium, with the exception of agar. In order to preserve the cells during freeze and thaw, thus maintaining its viability, 10% sterile glycerol was added to the C. *histolyticum* culture derived from the isolated colonies. This composition was then fractionated into cryotubes and frozen at -80 ºC for future use, such conditions allows the stock cultures to be used for at least 1 year without losing cell viability.
2 - Growth phase (pre-inoculum and inoculum preparation): a cell bank aliquot was rapidly thawed at room temperature ; a 100 µL aliquot was transferred to 1 mL of an animal product-free culture medium (Medium 1), to which 9 mL of the same medium was added for bacterial growth at 37 ºC under anaerobic conditions for about 16 hours (pre-inoculum). Subsequently, the pre-inoculums were used to generate the inoculums as described previously.
3 - Fermentation phase (production of supernatant of C. *histolyticum* liquid culture comprising proteases with collagenolytic and gelatinolytic activities): 60 mL of the inoculum were transferred to a fermenter containing 3 L of animal product-free culture medium (pH 7.0, Medium 1) and maintained at 37 ºC for about 14 hours under anaerobic conditions, through addition of nitrogen gas. During the culture, the pH was controlled via the addition of 5M NH₄OH when the pH reached values lower than 6.5, or 2MH₂SO₄ when the pH reached values higher than 8.0.
   The maximum bacterial growth rate and better proteases with collagenolytic and gelatinolytic activity production occurred after 14 hours of bacterial fermentation (Figure 10).The same process was carried out using Medium 6 as animal product-free culture medium described in step 3.
   Bacterial growth was monitored through optical density measurements (600 nm) which were performed using a spectrophotometer every 2 hours of culturing.
   The protein and enzymatic supernatant profile obtained from the *C. histolyticum* cultures described above were evaluated through gel electrophoresis (7.5% SDS PAGE) under non-reducing conditions and dyed with silver nitrate (Figure 11) and non-reducing SDS-PAGE comprising 10% gelatin and dyed with Coomassie blue (Figures 10 and 11; zymography).
4 - Obtainment of the supernatant of *C. histolyticum* liquid culture and proteases with collagenolytic and gelatinolytic activity recovery: In order to obtain the liquid culture supernatant and recover the collagenolytic and gelatinolytic enzymes it was followed the same procedure as the one described in Example 4 (centrifugation) and using saline precipitation followed by dialysis and lyophilization, according to the detailed description showed in Example 6, or tangential flow filtration (5 KDa pore size) followed by lyophilization. The collagenolytic and gelatinolytic activities of the supernatant were monitored through fluorimetric assays of detailed in Example 4 (results shown in Figures 10 and 11).

The results (OD, enzymatic activity and zymography) of two fermentative processes carried out in culture Medium 1 (Medium 1) are demonstrated in Figure 10

Figure 11 shows the results (OD, enzymatic activity, SDS-PAGE GEL/silver nitrate and zymography) of a fermentative process carried out in Medium 6. It can be observed in Figure 11, a bacterial growth curve similar to that obtained when the fermentation took place in a bioreactor using medium 1 (comprising cysteine only, Figure 10). However and surprisingly, it can also be observed a significantly higher collagenolytic and gelatinolytic activity, showing that the addition of arginine to the animal product-free culture medium influences the increase of collagenolytic and gelatinolytic enzymes production in *C. histolyticum* cultures (activity in U/mL).

Figure 12 shows the comparison of collagenolytic and gelatinolytic activity (mU/mL) and optical density (OD 600 nm) between the culture using Medium 1 (containing only amino acid cysteine) and the culture using Medium 6 (containing cysteine and arginine), clearly showing an increase on average of 30% of the collagenolytic and gelatinolytic activity yielded in a *C. histolyticum* culture supernatant. Additionally, images of SDS-PAGE dyed with silver nitrate and zymography of the supernatants obtained from the C. *histolyticum* cultures are presented in Figures 10 and 11, portraying the supernatant quality, predominantly having proteins with high molecular weight, identified here as being collagenases.

### Example 6. Recovery of proteases with collagenolytic and gelatinolytic activity obtained from a C. histolyticum liquid culture supernatant when cultured in an animal product-free culture medium.

The supernatant comprising the proteases with collagenolytic and gelatinolytic activity was obtained through the *C. histolyticum* culture according to Example 5, using Medium 6 (comprising cysteine and arginine) in step 3.

In order to retrieve the proteases present in the *C. histolyticum* liquid culture supernatant with collagenolytic and gelatinolytic activity, 243.73 g of (NH₄)₂SO₄ was added to 500 mL of supernatant, the mixture was then shaken and stored in a freezer for 2 h (saline precipitation stage). Subsequently, the mixture was centrifuged at a speed of 8500 rpm for 30 min at 4°C. The material, obtained after the saline precipitation and centrifugation, was resuspended in 12.0 mL of 2 mM sodium phosphate buffer, pH 7.2, and then submitted to dialysis with 2 mM sodium phosphate buffer, pH 7.2. Before the samples were frozen and lyophilized, there were three buffer changes. Lyophilization was carried out for at least 18 h. The lyophilized material was collected for analysis and characterization.

The dialyzed supernatant and the lyophilized material were characterized via 7.5% SDS-PAGE gel electrophoresis the samples were prepared under non-reducing conditions and stained with silver nitrate. Figure 3 represents the results acquired through the above mentioned procedure, demonstrating that the supernatant obtained through *C. histolyticum* culture in culture medium according to the present invention possesses an excellent quality. Not only, the proteases processed and lyophilized demonstrated high stability corroborating to the excelled supernatant quality. Through SDS-PAGE analysis it was possible to notice a predominant presence of high molecular weight proteins (collagenases), which prevalence is increased after lyophilization. The process described for the supernatant lyophilization shows a good output, since nearly 90% of the initial supernatant enzymatic activity was recovered.

### Example 7. Pharmaceutical composition for topical use comprising C. histolyticum collagenases - Stability Assays

The pharmaceutical composition used to exemplify the present invention was a dermatological ointment comprising the ingredients presented below:

| | Quantity/ g | | |
|---|---|---|---|
| Ingredient | Formulation 1 | Formulation 2 | Formulation 3 |
| Collagenase | 0.2 U | 0.6 U | 1.8 U |
| Liquid vaseline - mineral oil | 0.0188 mL | 0.0188 mL | 0.0188 mL |
| Solid vaseline - MBK | 0.9846 g | 0.9846 g | 0.9846 g |

The specific collagenase activity was determined by enzymatic hydrolysis of a synthetic substrate Carbobenzoxy-Gly-Pro-Gly-Gly-Pro-Ala, being classified through colorimetric assay with ninhydrin. An enzyme unit is defined as being responsible for the release of 1 µmol of Gly-Pro-Ala from Z-Gly-Pro-Gly-Gly-Pro-Ala hexapeptide in 1 minute at 37ºC.

The pharmaceutical composition was prepared through the dispersion of the lyophilized component (amount equivalent to the specific collagenase activity equal to 0.2 U, 0.6 U and 1.8 U/g of formulation), obtained according to Example 5 (lyophilized component of the supernatant obtained through a culture using Medium 6 after tangential flow filtration), in the other excipients and homogenized. The 0.6 U/g dose is considered a therapeutic dose proper for human use.

The stability study was carried out with formulation 2 described above, stored in aluminum tubes containing 30 g. The performed tests, specification and observed results are described in Tables 3 and 4. All stability assays were performed in environmental chambers specific for stability assays.

**Table 3. Long term stability: 30°C ± 2°C, 75% ± 5% UR (Relative Humidity).**

| **TEST** | **SPECIFICATION** | **ANALYSIS PERIODS** | | |
|---|---|---|---|---|
| | | **INITIAL** | **3 months** | **6 months** |
| **ASPECT** | SOFT, FOREIGN PARTICLES AND LUMP FREE OINTMENT | IN ACCORDANCE | IN ACCORDANCE | IN ACCORDANCE |
| **COLOR** | WHITE TO LIGHT BROWN | IN ACCORDANCE | IN ACCORDANCE | IN ACCORDANCE |
| **ODOR** | DISTINCTIVE | IN ACCORDANCE | IN ACCORDANCE | IN ACCORDANCE |
| **COLLAGENASE CONTENT** | BETWEEN 90.0 - 120.0% | 106.5% | 103.3% | 107.4% |
| | 0.54 - 0.72 U/g | 0.64 U/g | 0.62 U/g | 0.64 U/g |

**Table 4. Accelerated stability: 40°C ± 2°C, 75% ± 5% UR (Relative Humidity).**

| **TEST** | **SPECIFICATION** | **ANALYSIS PERIODS** | | |
|---|---|---|---|---|
| | | **INITIAL** | **3 months** | **6 months** |
| **ASPECT** | SOFT, FOREIGN PARTICLES AND LUMP FREE OINTMENT | IN ACCORDANCE | IN ACCORDANCE | IN ACCORDANCE |
| **COLOR** | WHITE TO LIGHT BROWN | IN ACCORDANCE | IN ACCORDANCE | IN ACCORDANCE |
| **ODOR** | DISTINCTIVE | IN ACCORDANCE | IN ACCORDANCE | IN ACCORDANCE |
| **COLLAGENASE CONTENT** | BETWEEN 90.0 - 120.0% | 106.5% | 104.5% | 109% |
| | 0.54 - 0.72U/g | 0.64 U/g | 0.63 U/g | 0.65 U/g |

It can be observed the characteristics maintenance of the initial product in both evaluated conditions, confirming the product quality obtained through culture Medium 6 (comprising cysteine and arginine) and process for obtaining a supernatant from *Clostridium histolyticum* liquid culture described in the present invention.

### Example 8. Pharmaceutical composition for topic use comprising C. histolyticum collagenases - Toxicity

The pharmaceutical compositions used to exemplify the present invention are the same used to perform stability assays described above (Example 7).

It was performed a dermal toxicity assay with repeated doses (21 days) on rats scarified skin with the prepared compositions according to Example 7 (active ingredient: lyophilized component with specific Collagenase activity of 0.2 U/g, 0.6 U/g and 1.8 U/g). As an assay control, it was used a placebo composition, prepared according to Example 7, however, without adding the active ingredient.

The assay, based on the OECD 410 Guideline, consisted in surgically provoking an injury using a 4mm sterile punch at the center of the dorsal median line on the first day of application (dorsal area hair removed by an epilator device). A topical application of the composition (0.425g; compositions containing 0.2 U/g, 0.6 U/g and 1.8 U/g of collagenase), to an area equivalent to 30-40 cm², was done with a spatula, inside and around the injury, once-a-day, for 21 days. This was performed in three groups of *Rattus norvegicus* (albino variety, Wistar lineage) in which there were 10 rats per group, 5 males and 5 nulliparous and non-pregnant females.

The application site was preserved with gauze and a nonirritating tape for about 24 hours after the application.

In parallel, to a control group composed of 10 rats, 5 males and 5 females, only placebo was applied (Collagenase Placebo). Additionally, two satellite groups were formed, one evaluated with a higher dose of the test composition (10 rats, 5 males and 5 females) and another with the control composition (10 rats, 5 males and 5 females). They were also treated for 21 days and monitored for 14 days after the final period of exposure, with the intent of verifying reversibility, persistence or late effects of exposure to a test substance.

The following parameters were daily observed after each application:
1. Changes in hair and skin.
2. Changes in the eyes and mucous membranes.
3. Changes in breathing and circulation.
4. Changes in the Autonomous and Central Nervous System.
5. Changes in somatic-motor activity and standard behavior.
6. Other changes like trembling, seizure, salivation, diarrhea, lethargy, sleepiness and coma.

The rats' weight (Table 5), and water and food consumption were recorded weekly. At the end of the study, blood samples were collected for hematological analysis (hematocrit, hemoglobin concentration, erythrocytes count, red blood cell indexes calculation, total and differential leukocyte, prothrombin time, activated partial thromboplastin time and platelets count - Table 6) and biochemical (sodium, potassium, calcium, phosphorus, triglycerides, albumin, creatinine, urea, total protein, alanine and aspartate aminotransferases, cholesterol, and glycemia - Table 7). Later, the animals were submitted to euthanasia and necropsy (Table 8). Tissue samples were forwarded to histopathological analysis.

The application of repeated doses (21 days) of the described pharmaceutical composition, in all evaluated doses, did not induce death, nor changes in clinical evaluations such as body weight, food and water consumption, hematological, biochemical and/or anatomopathological tests all which could possibly be associated with the exposure to the active ingredient. Thus, the obtained results demonstrated that the application of the pharmaceutical composition comprising the lyophilized component (amount equivalent to the specific collagenase activity equal to 0.2 U, 0.6 U and 1.8 U/g formulation), obtained according to Example 5 (supernatant lyophilized component obtained after tangential flow filtration procedure) over a period of 21 days via dermal exposure to *Rattus novergicus* in the 0.2 U/g, 0.6 U/g and 1.8 U/g formulations, was well tolerated and did not provoke changes possibly related to toxicity. It may surprisingly be observed that even with a dose therapeutic three-times superior than that considered for humans, the described composition in the present invention does not show signs of toxicity in rats.

**Table 5. Body weight (male and female rats). Trial groups for the evaluation of dermal toxicity through repeated doses (21 days) of topical composition comprising purified supernatant and lyophilized component derived from C. histolyticum culture (ATCC 21000) using an animal product-free culture medium comprising soy peptone (30 g/L), yeast extract (30 g/L), cysteine hydrochloride (0.625 g/L) and arginine hydrochloride (3.75 g/L). Control composition: only with excipients.**

| | **Groups- males** | | | | | | | | |
|---|---|---|---|---|---|---|---|---|---|
| | 0.2 U/g | | | 0.6 U/g | | | 1.8 U/g | | |
| **WEEKS** | **MEAN** | **S.D** | **n** | **MEAN** | **S.D** | **n** | **MEAN** | **S.D** | **n** |
| **0** | 296.40 | 22.56 | 5 | 302.12 | 26.92 | 5 | 306.56 | 13.94 | 5 |
| **1** | 279.14 | 17.93 | 5 | 282.68 | 28.46 | 5 | 292.72 | 19.68 | 5 |
| **2** | 283.04 | 17.54 | 5 | 277.10 | 15.17 | 5 | 301.68 | 15.09 | 5 |
| **3** | 280.94 | 19.34 | 5 | 273.42 | 18.43 | 5 | 291.20 | 20.19 | 5 |
| **4** | | | | | | | | | |
| **5** | | | | | | | | | |

| | | **Groups - males** | | | | | | | |
|---|---|---|---|---|---|---|---|---|---|
| | Control | | | Satellite-Control | | | 1.8 U/g-Satellite | | |
| **WEEKS** | **MEAN** | **S.D** | **n** | **MEAN** | **S.D.** | **n** | **MEAN** | **S.D.** | **n** |
| **0** | 298.68 | 14.04 | 5 | 284.00 | 39.46 | 5 | 311.18 | 18.05 | 5 |
| **1** | 279.42 | 15.00 | 5 | 285.16 | 15.10 | 5 | 292.58 | 15.10 | 5 |
| **2** | 287.22 | 19.90 | 5 | 293.50 | 17.76 | 5 | 295.92 | 14.75 | 5 |
| **3** | 278.96 | 16.01 | 5 | 298.08 | 16.36 | 5 | 300.94 | 15.77 | 5 |
| **4** | | | | 328.12 | 9.95 | 7 | 335.12 | 21.56 | 5 |
| **5** | 324.26 | 324.26 | 324.26 | 324.26 | 9.24 | 5 | 338.34 | 17.92 | 5 |

| | **Groups -females** | | | | | | | | |
|---|---|---|---|---|---|---|---|---|---|
| | **0.2 U/g** | | | **0.6** U/g | | | **1.8 U/g** | | |
| **WEEKS** | **MEAN** | **S.D** | **n** | **MEAN** | **S.D.** | **n** | **MEAN** | **S.D.** | **n** |
| **0** | 252.20 | 21.28 | 5 | 221.00 | 7.89 | 5 | 238.86 | 14.88 | 5 |
| **1** | 238.02 | 21.38 | 5 | 215.14 | 15.93 | 5 | 226.60 | 10.31 | 5 |
| **2** | 247.14 | 21.54 | 5 | 218.36 | 10.92 | 5 | 235.10 | 12.05 | 5 |
| **3** | 244.42 | 15.77 | 5 | 212.52 | 12.20 | 5 | 240.98 | 12.59 | 5 |
| **4** | | | | | | | | | |
| **5** | | | | | | | | | |

| | **Groups -females** | | | | | | | | |
|---|---|---|---|---|---|---|---|---|---|
| | **Control** | | | **Satellite-Control** | | | **1.8 U/g-Satellite** | | |
| **WEEKS** | **MEAN** | **S.D n** | | **MEAN** | | **n** | **MEAN** | **S.D.** | **n** |
| **0** | 230.56 | 19.88 | 5 | 252.72 | 32.44 | 5 | 234.16 | 8.57 | 5 |
| **1** | 223.60 | 9.98 | 5 | 228.32 | 11.05 | 5 | 226.70 | 9.75 | 5 |
| **2** | 235.72 | 15.04 | 5 | 238.44 | 7.61 | 7 | 227.64 | 8.16 | 5 |
| **3** | 225.42 | 13.91 | 5 | 247.70 | 15.98 | 5 | 242.98 | 11.99 | 5 |
| **4** | | | | 258.32 | 12.35 | 5 | 256.58 | 9.85 | 5 |
| **5** | | | | 247.16 | 6.91 | 5 | 247.12 | 7.71 | 5 |

**Table 6. Hematological analysis (male and female rats). Trial groups for the evaluation of dermal toxicity through repeated doses (21 days) of topical composition comprising purified supernatant and lyophilized derived from C. histolyticum culture (ATCC 21000) in animal product-free culture medium comprising soy peptone (30 g/L), yeast extract (30 g/L), cysteine hydrochloride (0.625 g/L) and arginine hydrochloride (3.75 g/L). Control composition: only with excipients.**

| | **Groups - males** | | | | | | | | |
|---|---|---|---|---|---|---|---|---|---|
| | **0.2 U/g** | | | **0.6 U/g** | | | **1.8 U/g** | | |
| | **MEAN** | **S.D.** | **n** | **MEAN** | **S.D**. | **n** | **MEAN** | **S.D.** | **n** |
| **Erythrocyte** mm³ | 9.68 | 0.50 | 5 | 9.46 | 0.17 | 5 | 9.26 | 0.53 | 5 |
| **Hematocrit** % | 54.20 | 2.17 | 5 | 53.20 | 0.84 | 5 | 53.00 | 2.12 | 5 |
| **Hemoglobin** g/dL | 19.06 | 0.61 | 5 | 18.62 | 0.37 | 5 | 18.62 | 1.03 | 5 |
| MCV fL | 56.02 | 1.01 | 5 | 56.25 | 1.50 | 5 | 57.31 | 2.18 | 5 |
| MCH Pg | 19.71 | 0.60 | 5 | 19.69 | 0.64 | 5 | 20.11 | 0.32 | 5 |
| MCHC mg/dL | 35.18 | 0.69 | 5 | 35.00 | 0.74 | 5 | 35.12 | 0.93 | |
| **Leucocyte** mm³ | 6060.00 | 1145.86 | 5 | 6700.00 | 1208.30 | 5 | 6140.00 | 1335.29 | 5 |
| **Segmented** mm³ | 2017.00 | 780.36 | 5 | 1910.00 | 661.28 | 5 | 1698.20 | 659.76 | 5 |
| **Eosinophil** mm³ | 59.60 | 36.16 | 5 | 28.00 | 38.66 | 5 | 43.80 | 66.46 | 5 |
| Lymphocyte mm³ | 3864.00 | 842.49 | 5 | 4651.80 | 711.06 | 5 | 4292.80 | 860.02 | 5 |
| Monocyte mm³ | 119.40 | 38.86 | 5 | 125.60 | 56.70 | 5 | 105.20 | 76.67 | 5 |
| **Platelets** mm³ | 1164600.0 | 111428.5 | 5 | 1276200.0 | 174246.1 | 5 | 1207800.0 | 207705.8 | 5 |
| P T Seg | 8.00 | 0.00 | 5 | 8.20 | 0.45 | 5 | 8.60 | 0.55 | 5 |
| Pa T T Seg | 29.80 | 1.10 | 5 | 30.20 | 0.84 | 5 | 30.40 | 1.14 | 5 |

| | **Groups - males** | | | | | | | | |
|---|---|---|---|---|---|---|---|---|---|
| | **Control** | | | **Satellite-Control** | | | **1.8 U/g-Satellite** | | |
| | **MEAN** | | **n** | **MEAN** | **S.D.** | **n** | **MEAN** | **S.D.** | **n** |
| **Erythrocyte** mm³ | 9.32 | 0.26 | 5 | 9.34 | 0.34 | 5 | 9.30 | 0.20 | 5 |
| **Hematocrit** % | 51.60 | 1.52 | 5 | 51.40 | 2.61 | 5 | 52.00 | 2.74 | 5 |
| **Hemoglobin** g/dL | 17.88 | 0.55 | 5 | 18.30 | 0.62 | 5 | 18.26 | 1.04 | 5 |
| MCV fL | 55.38 | 1.39 | 5 | 55.03 | 1.80 | 5 | 55.89 | 2.00 | 5 |
| MCH Pg | 19.18 | 0.26 | 5 | 19.60 | 0.45 | 5 | 19.62 | 0.74 | 5 |
| MCHC mg/dL | 34.66 | 0.72 | 5 | 35.63 | 0.70 | 5 | 35.12 | 0.70 | 5 |
| **Leucocyte** mm³ | 5900.00 | 1449.14 | 5 | 5520.00 | 884.31 | 5 | 6360.00 | 1099.09 | 5 |
| **Segmented** mm³ | 1824.20 | 986.49 | 5 | 1262.80 | 408.65 | 5 | 1712.40 | 659.01 | 5 |
| **Eosinophil** mm³ | 38.00 | 36.99 | 5 | 61.80 | 48.48 | 5 | 28.40 | 39.12 | 5 |
| Lymphocyte mm³ | 3946.60 | 661.89 | 5 | 4139.20 | 731.73 | 5 | 4593.20 | 1082.69 | 5 |
| Monocyte mm³ | 91.20 | 30.55 | 5 | 77.00 | 30.67 | 5 | 26.00 | 36.60 | 5 |
| **Platelets** mm³ | 1078000.0 | 112712.0 | 5 | 983400.0 | 91971.73 | 5 | 1142600.0 | 210515.6 | 5 |
| P T Seg | 8.00 | 0.00 | 4 | 8.00 | 0.00 | 5 | 8.60 | 0.55 | 5 |
| Pa T T Seg | 29.50 | 0.58 | 4 | 28.20 | 1.30 | 5 | 28.60 | 1.14 | 5 |

| | **Groups - females** | | | | | | | | |
|---|---|---|---|---|---|---|---|---|---|
| | **0.2 U/g** | | | **0.6 U/g** | | | **1.8 U/g** | | |
| | **MEAN** | **S.D**. | **n** | **MEAN** | **S.D.** | **n** | **MEAN** | **S.D.** | **n** |
| **Erythrocyte** mm³ | 8.14 | 0.46 | 5 | 8.32 | 0.55 | 5 | 7.80 | 0.42 | 5 |
| **Hematocrit %** | 49.00 | 1.41 | 5 | 49.40 | 2.70 | 5 | 47.00 | 1.22 | 5 |
| **Hemoglobin** g/dL | 17.10 | 0.66 | 5 | 16. 80 | 0.93 | 5 | 16.18 | 0.76 | 5 |
| MCV fL | 60.28 | 1.86 | 5 | 59.44 | 2.32 | 5 | 60.39 | 3.44 | 5 |
| MCH Pg | 21.04 | 0.98 | 5 | 20.21 | 0.50 | 5 | 20.77 | 0.93 | 5 |
| MCHC mg/dL | 34.90 | 0.94 | 5 | 34.02 | 0.86 | 5 | 34.42 | 1.28 | 5 |
| **Leucocyte** mm³ | 4860.00 | 1062.07 | 5 | 4880.00 | 846.76 | 5 | 5020.00 | 1551.45 | 5 |
| **Segmented** mm³ | 1364.80 | 402.06 | 5 | 1123.00 | 332.24 | 5 | 1328.00 | 437.38 | 5 |
| **Eosinophil** mm³ | 52.20 | 45.37 | 5 | 12.40 | 27.73 | 5 | 9.40 | 21.02 | 5 |
| Lymphocyte mm³ | 3382.00 | 693.01 | 5 | 3659.00 | 646.94 | 5 | 3625.80 | 1134.61 | 5 |
| Monocyte mm³ | 61.00 | 36.01 | 5 | 85.60 | 34.67 | 5 | 56.80 | 13.22 | 5 |
| **Platelets** mm³ | 1132400 | 222246.0 | 5 | 1114600.0 | 91876.55 | | 1011000.0 | 148403.2 | |
| P T Seg | 8.40 | 0.89 | 5 | 8.00 | 0.00 | 5 | 8.40 | 0.89 | 5 |
| Pa T T Seg | 30.60 | 1.14 | 5 | 30.00 | 0.71 | 5 | 30.00 | 0,71 | 5 |

| | **Groups - females** | | | | | | | | |
|---|---|---|---|---|---|---|---|---|---|
| | **Control** | | | **Satellite-Control** | | | **1,8 U/g-Satellite** | | |
| | **MEAN** | **S.D.** | **n** | **MEAN** | **S.D.** | **n** | **MEAN** | **S.D.** | **n** |
| **Erythrocyte** mm³ | 8.36 | 0.61 | 5 | 8.76 | 0.41 | 5 | 8.60 | 0.42 | 5 |
| **Hematocrit** % | 48.20 | 1.48 | 5 | 50.40 | 2.07 | 5 | 49.00 | 0.71 | 5 |
| **Hemoglobin** g/dL | 17.04 | 0.44 | 5 | 17.82 | 1.07 | 5 | 17.62 | 0.50 | 5 |
| MCV fL | 57.82 | 3.12 | 5 | 57.55 | 0.96 | 5 | 57.10 | 3.21 | 5 |
| MCH Pg | 20.45 | 1.31 | 5 | 20.33 | 0.43 | 5 | 20.54 | 1.35 | 5 |
| MCHC mg/dL | 35.36 | 0.71 | 5 | 35.34 | 1.04 | 5 | 35.96 | 0.85 | 5 |
| **Leucocyte** mm³ | 5180.0 | 1377.32 | 5 | 4440.00 | 1078.42 | 5 | 5280.00 | 1637.68 | 5 |
| **Segmented** | 1386.0 | 450.85 | 5 | 815.60 | 439.77 | 5 | 995.60 | 415.46 | 5 |
| **Eosinophil** mm³ | 27.60 | 26.14 | 5 | 65.20 | 67.48 | 5 | 43.00 | 29.01 | 5 |
| Lymphocyte mm³ | 3661.6 | 996.95 | 5 | 3497.20 | 783.17 | 5 | 4127.20 | 1452.48 | 5 |
| Monocyte mm³ | 104.8 | 95.46 | 5 | 62.00 | 44.32 | 5 | 114.20 | 71.51 | 5 |
| **Platelets** mm³ | 1050600 | 200655.9 | 5 | 1002000.0 | 86743.88 | 5 | 1028400.0 | 33080.21 | 5 |
| P T Seg | 8.20 | 0.45 | 5 | 8.20 | 0.84 | 5 | 8.40 | 0.55 | 5 |
| Pa T T Seg | 30.00 | 0.00 | 5 | 28.60 | 0.55 | 5 | 29.00 | 1.00 | 5 |

**Table 7. Biochemical analysis (male and female rats). Trial groups for the evaluation of dermal toxicity through repeated doses (21 days) of topical composition comprising purified supernatant and lyophilized derived from C. histolyticum culture (ATCC 21000) in animal product-free culture medium comprising soy peptone (30 g/L), yeast extract (30 g/L), cysteine hydrochloride (0.625 g/L) and arginine hydrochloride (3.75 g/L). Control composition: only with excipients.**

| | **Groups - males** | | | | | | | | |
|---|---|---|---|---|---|---|---|---|---|
| | **0.2 U/g** | | | **0.6 U/g** | | | **1.8 U/g** | | |
| | **MEAN** | **S.D.** | **n** | **MEAN** | **S.D.** | **n** | **MEAN** | **S.D.** | **n** |
| **Albumin** g/dl | 4.58 | 0.13 | 5 | 4.46 | 0.15 | 5 | 4.60 | 0.25 | 5 |
| **ALT** UI/L | 67.86 | 8.03 | 5 | 58.92 | 5.05 | 5 | 62.00 | 8.86 | 5 |
| **AST** UI/L | 143.66 | 14.39 | 5 | 130.38 | 18.78 | 5 | 138.00 | 22.03 | 5 |
| **Calcium** mg/dl | 10.46 | 0.54 | 5 | 10.34 | 0.46 | 5 | 10.68 | 0.49 | 5 |
| **Total Cholesterol** mg/dl | 57.16 | 2.56 | 5 | 64.74 | 9.64 | 5 | 56.74 | 4.61 | 5 |
| **Creatinine** mg/dl | 0.36 | 0.05 | 5 | 0.32 | 0.08 | 5 | 0.36 | 0.05 | 5 |
| **Phosphorus** mg/dl | 7.84 | 0.65 | 5 | 7.44 | 0.54 | 5 | 8.30 | 0.80 | 5 |
| **Glucose** mg/dl | 96.00 | 9.43 | 5 | 97.60 | 12.18 | 5 | 93.60 | 11.28 | 5 |
| **Potassium** mEq/L | 6.20 | 0.66 | 5 | 5.24 | 0.49 | 5 | 5.88 | 0.63 | 5 |
| Total Proteins g/dl | 5.80 | 0.19 | 5 | 5.74 | 0.23 | 5 | 5.90 | 0.19 | 5 |
| **Sodium** mEq/L | 141.40 | 3.36 | 5 | 142.60 | 0.89 | 5 | 141.20 | 3.35 | 5 |
| **Triglycerides** mg/dl | 39.04 | 5.66 | 5 | 35.86 | 6.34 | 5 | 34.78 | 6.36 | 5 |
| **Urea** mg/dl | 57.62 | 8.41 | 5 | 60.16 | 7.49 | 5 | 58.56 | 1.78 | 5 |

| | **Groups - males** | | | | | | | | |
|---|---|---|---|---|---|---|---|---|---|
| | **Control** | | | **Satellite-Control** | | | **1,8 U/g-Satellite** | | |
| | **MEAN** | **S.D.** | **n** | **MEAN** | **S.D** | **n** | **MEAN** | **D.P.** | **n** |
| **Albumin** g/ dl | 4.46 | 0.13 | 5 | 4.70 | 0.16 | 5 | 4.72 | 0.22 | 5 |
| **ALT** UI/L | 63.80 | 16.36 | 5 | 53.36 | 9.38 | 5 | 54.14 | 13.51 | 5 |
| **AST** UI/L | 155.12 | 18.16 | | 115.00 | 16.16 | 5 | 112.18 | 30.71 | 5 |
| **Calcium** mg/dl | 10.38 | 0.29 | 5 | 10.70 | 0.57 | 5 | 10.68 | 0.25 | 5 |
| **Total Cholesterol** mg/dl | 61.72 | 7.43 | 5 | 59.52 | 6.40 | 5 | 69.50 | 14.49 | 5 |
| **Creatinine** mg/dl | 0.38 | 0.04 | 5 | 0.42 | 0.04 | 5 | 0.36 | 0.05 | 5 |
| **Phosphorus** mg/dl | 8.22 | 0.72 | 5 | 7.54 | 0.77 | 5 | 7.88 | 0.69 | 5 |
| **Glucose** mg/dl | 88.20 | 8.29 | 5 | 106.80 | 24.24 | 5 | 104.20 | 7.89 | 5 |
| **Potassium** mEq/L | 7.00 | 1.14 | 5 | 6.12 | 0.29 | 5 | 6.32 | 0.32 | 5 |
| Total Proteins g/dl | 5.66 | 0.30 | 5 | 6.30 | 0.16 | 5 | 6.42 | 0.33 | 5 |
| **Sodium** mEq/L | 158.00 | 27.40 | 5 | 142.60 | 1.14 | 5 | 141.60 | 1.82 | 5 |
| **Triglycerides** mg/dl | 43.30 | 7.58 | 5 | 57.66 | 9.18 | 5 | 67.49 | 46.41 | 5 |
| **Urea** mg/dl | 60.86 | 2.29 | 5 | 56.58 | 6.25 | 5 | 56.20 | 4,26 | 5 |

| | **Groups - females** | | | | | | | | |
|---|---|---|---|---|---|---|---|---|---|
| | **0.2 U/g** | | | **0.6 U/g** | | | **1.8 U/g** | | |
| | **MEAN** | **S.D.** | **n** | **MEAN** | **S.D**: | **n** | **MEAN** | **S.D.** | **n** |
| **Albumin** g/dl | 4.70 | 0.17 | 5 | 4.44 | 0.17 | 5 | 4.64 | 0.11 | 5 |
| **ALT** UI/L | 66.46 | 15.07 | 5 | 69.38 | 14.48 | 5 | 61.56 | 8.22 | 5 |
| **AST** UI/L | 150.92 | 17.67 | 5 | 184.06 | 50.29 | 5 | 158.72 | 26.90 | 5 |
| **Calcium** mg/dl | 10.80 80 | 0.71 | 5 | 10.32 | 0.43 | 5 | 10.62 | 0.35 | 5 |
| **Total Cholesterol** mg/dl | 53.68 | 11.37 | 5 | 47.74 | 8.86 | 5 | 48.32 | 8.44 | 5 |
| **Creatinine** mg/dl | 0.38 | 0.08 | 5 | 0.42 | 0.08 | 5 | 0.30 | 0.07 | 5 |
| **Phosphorus** mg/dl | 7.66 | 1.28 | 5 | 8.24 | 1.75 | 5 | 8.06 | 1.85 | 5 |
| **Glucose** mg/dl | 90.80 | 12.38 | 5 | 93.60 | 12.05 | 5 | 95.20 | 20.03 | 5 |
| **Potassium** mEq/L | 6.04 | 0.87 | 5 | 6.08 | 1.26 | 5 | 7.20 | 2.07 | 5 |
| Total Proteins g/dl | 6.02 | 0.19 | 5 | 5.90 | 0.07 | 5 | 5.96 | 0.13 | 5 |
| **Sodium** mEq/L | 154.40 | 25.52 | 5 | 142.20 | 1.30 | 5 | 166.40 | 29.07 | 5 |
| **Triglycerides** mg/dl | 39.34 | 7.38 | 5 | 33.08 | 3.88 | 5 | 44.84 | 13.32 | 5 |
| **Urea** mg/dl | 57.42 | 3.85 | 5 | 63.54 | 7.95 | 5 | 57.02 | 12.79 | 5 |

| | **Groups - females** | | | | | | | | |
|---|---|---|---|---|---|---|---|---|---|
| | **Control** | | | **Satellite-Control** | | | **1,8 U/g-Satellite** | | |
| | **MEAN** | **S.D**. | **n** | **MEAN** | **S.D.** | **n** | **MEAN** | **S.D.** | **n** |
| **Albumin** g/dl | 4.54 | 0.15 | 5 | 5.04 | 0.29 | 5 | 4.84 | 0.18 | 5 |
| **ALT** U I/L | 49.04 | 9.68 | 5 | 37.38 | 5.90 | 5 | 41.88 | 15.63 | 5 |
| **AST** U I/L | 173.02 | 28.78 | 5 | 97.82 | 9.16 | 5 | 124.84 | 17.31 | 5 |
| **Calcium** mg/dl | 10.33 | 0.34 | 5 | 10.64 | 0.25 | 5 | 10.64 | 0.21 | 5 |
| **Total Cholesterol** mg/dl | 39.74 | 3.50 | 5 | 43.70 | 14.02 | 5 | 36.15 | 7.24 | 5 |
| **Creatinine** mg/dl | 0.36 | 0.05 | 5 | 0.44 | 0.09 | 5 | 0.42 | 0.04 | 5 |
| **Phosphorus** mg/dl | 7.70 | 0.84 | 5 | 6.06 | 0.30 | 5 | 7.16 | 0.48 | 5 |
| **Glucose** mg/dl | 89.80 | 13.29 | 5 | 110.40 | 13.46 | 5 | 100.40 | 15.50 | 5 |
| **Potassium** mEq/L | 5.92 | 0.33 | 5 | 5.60 | 0.35 | 5 | 6.18 | 0.46 | 5 |
| Total Proteins g/dl | 5.76 | 0.30 | 5 | 6.74 | 0.13 | 5 | 6.54 | 0.23 | 5 |
| **Sodium** mEq/L | 141.20 | 4.44 | 5 | 142.60 | 1.14 | 5 | 142.60 | 0.89 | 5 |
| **Triglycerides** mg/ dl | 35.14 | 4.26 | 5 | 46.76 | 11.39 | 5 | 45.54 | 4.70 | 5 |
| **Urea** mg/dl | 56.74 | 3.94 | 5 | 52.90 | 4.48 | 5 | 54.26 | 4.90 | 5 |

**Table 8. Organs weight analysis (male and female rats). Trial groups for the evaluation of dermal toxicity through repeated doses (21 days) of topical composition comprising purified supernatant and lyophilized derived from C. histolyticum culture (ATCC 21000) in animal product-free culture medium comprising soy peptone (30 g/L), yeast extract (30 g/L), cysteine hydrochloride (0.625 g/L) and arginine hydrochloride (3.75 g/L). Control composition: only with excipients.**

| | **Groups - males** | | | | | | | | |
|---|---|---|---|---|---|---|---|---|---|
| | **0.2 U/g** | | | **0.6 U/g** | | | **1.8 U/g** | | |
| | **MEAN** | **S.D.** | **n** | **MEAN** | **S.D.** | **n** | **MEAN** | **S.D.** | **n** |
| Liver Absolute (g) | 9.4932 | 0.7372 | 5 | 9.2295 | 0.7889 | 5 | 9.7103 | 0.9560 | 5 |
| Liver Relative | 0.0338 | 0.0022 | 5 | 0.0338 | 0.0023 | 5 | 0.0333 | 0.0010 | 5 |
| Kidneys Absolute (g) | 2.4209 | 0.0849 | 5 | 2.2266 | 0.1520 | 5 | 2.4551 | 0.2097 | 5 |
| Kidneys Relative | 0.0086 | 0.0005 | 5 | 0.0082 | 0.0004 | 5 | 0.0084 | 0.0003 | 5 |
| Adrenal Absolute (g) | 0.1014 | 0.0078 | 5 | 0.0968 | 0.0144 | 5 | 0.1026 | 0.0108 | 5 |
| Adrenal Relative | 0004 | 0.0000 | 5 | 0.0004 | 0.0001 | 5 | 0.0004 | 0.0000 | 5 |
| Testicles Absolute. | 3.5685 | 0.2740 | 5 | 3.1856 | 0.3267 | 5 | 3.4118 | 0.0909 | 5 |
| Testicles Relative | 0.0127 | 0.0010 | 5 | 0.0116 | 0.0005 | 5 | 0.0118 | 0.0007 | 5 |
| Test-Systems (g) | 280.94 | 19.34 | 5 | 273.42 | 18.43 | 5 | 291.20 | 20.19 | 5 |

| | **Groups** - **males** | | | | | | | | |
|---|---|---|---|---|---|---|---|---|---|
| | **Control** | | | **Satellite-Control** | | | **1.8 U/g-Satellite** | | |
| | **MEAN** | **S.D.** | **n** | **MEAN** | **S.D.** | **n** | **MEAN** | **S.D.** | **n** |
| Liver Absolute (g) | 9.0665 | 0.6492 | 5 | 10.4859 | 1.0222 | 5 | 11.7093 | 0.8423 | 5 |
| Liver Relative | 0.0325 | 0.0013 | 5 | 0.0323 | 0.0024 | 5 | 0.0346 | 0.0010 | 5 |
| Kidneys Absolute (g) | 2.2151 | 0.0912 | 5 | 2.6912 | 0.2611 | 5 | 2.6810 | 0.2522 | 5 |
| Kidneys Relative | 0.0080 | 0.0004 | 5 | 0.0083 | 0.0006 | 5 | 0.0079 | 0.0009 | 5 |
| Adrenal Absolute (g) | 0.0919 | 0.0124 | 5 | 0.1212 | 0.0120 | 5 | 0.1168 | 0.0085 | 5 |
| Adrenal Relative | 0.0003 | 0.0000 | 5 | 0.0004 | 0.0000 | 5 | 0.0003 | 0.0000 | 5 |
| Testicles Absolute. | 3.1967 | 0.1139 | 5 | 3.5597 | 0.2256 | 5 | 3.5676 | 0.3147 | |
| Testicles Relative | 0.0115 | 0.0007 | 5 | 0.0110 | 0.0007 | 5 | 0.0106 | 0.0011 | 5 |
| Test-Systems (g) | 278.96 | 16.01 | 5 | 324.26 | 9.24 | 5 | 338.34 | 17.92 | 5 |

| | **Groups - females** | | | | | | | | |
|---|---|---|---|---|---|---|---|---|---|
| | **0.2 U/g** | | | **0.6 U/g** | | | **1.8 U/g** | | |
| | **MEAN** | **S.D.** | **n** | **MEAN** | **S.D.** | **n** | **MEAN** | **S.D.** | **n** |
| Liver Absolute (g) | 8.5915 | 0.4047 | 5 | 7.3860 | 0.8336 | 5 | 9.0608 | 0.1947 | 5 |
| Liver Relative | 0.0352 | 0.0017 | 5 | 0.0347 | 0.0028 | 5 | 0.0377 | 0.0015 | 5 |
| Kidneys | 1.9906 | 0.1441 | 5 | 1.7287 | 0.1963 | 5 | 1.9488 | 0.0750 | 5 |
| Relative | 0.0082 | 0.0009 | 5 | 0.0081 | 0.0007 | 5 | 0.0081 | 0.0003 | 5 |
| Adrenal Absolute (g) | 0.1230 | 0.0139 | 5 | 0.1155 | 0.0189 | 5 | 0.1180 | 0.0201 | 5 |
| Adrenal Relative | 0.0005 | 0.0001 | 5 | 0.0005 | 0.0001 | 5 | 0.0005 | 0.0001 | 5 |
| Test-systems (g) | 244.42 | 15.77 | 5 | 212.52 | 12.20 | 5 | 240.98 | 12.59 | 5 |

| | **Control** | | | **Satellite-Control** | | | **1.8 U/g-Satellite** | | |
|---|---|---|---|---|---|---|---|---|---|
| | **MEAN** | **S.D.** | **n** | **MEAN** | **S.D.** | | **MEAN** | **S.D.** | **n** |
| Liver Absolute (g) | 7.4838 | 0.3414 | 5 | 7.8360 | 0.3740 | 5 | 7.7555 | 0.5574 | 5 |
| Liver Relative | 0.0332 | 0.0007 | 5 | 0.0317 | 0.0010 | 5 | 0.0314 | 0.0018 | 5 |
| Kidneys Absolute (g) | 1.8299 | 0.0613 | 5 | 1.9183 | 0.1122 | 5 | 1.9155 | 0.1215 | 5 |
| Kidneys Relative | 0.0081 | 0.0005 | 5 | 0.0078 | 0.0005 | 5 | 0. 0078 | 0.0005 | 5 |
| Adrenal Absolute (g) | 0.1008 | 0.0071 | 5 | 0.1257 | 0.0184 | 5 | 0.1211 | 0.0158 | 5 |
| Adrenal Relative | 0.0004 | 0.0000 | 5 | 0.0005 | 0.0001 | 5 | 0.0005 | 0.0001 | 5 |
| Test-systems (g) | 225.42 | 13.91 | 5 | 247.16 | 6.91 | 5 | 247.12 | 7.71 | 5 |

### Example 9. Production of supernatant of C. histolyticum liquid culture (ATCC 21000 and T248 strains) comprising proteases with collagenolytic and gelatinolytic activities in animal product-free culture medium - bioreactors culture.

The bioreactors cultures for evaluated supernatants production and the analytical methods were carried out as previously described in Example 5, using Medium 6 (comprising cysteine and arginine) as medium in step 3.
**Figure 14** shows a comparative analysis of the collagenolytic and gelatinolytic activities (mU/mL) and optical density (OD 600 nm) between *Clostridium histolyticum* ATCC 21000 and T248 cultures. There is no significant difference (statistical analysis: Mann Whitney, p <0.05) between the collagenolytic and gelatinolytic activity of the supernatants obtained from the culture of both *C. histolyticum* strains.

### Example 10. Cytotoxicity test of collagenolytic and gelatinolytic proteases obtained from Clostridium histolyticum (ATCC 21000 e T248) liquid culture supernatant using an animal product-free culture medium.

The collagenolytic and gelatinolytic proteases were obtained according to Example 6 (lyophilize samples).

The cells used in the cytotoxicity test were of fibroblastic type, from a lineage established in V79 clone M-8 cultures from Chinese Hamster (*Cricetulus griseus*) lung. The fibroblasts were maintained in 25cm² culture flasks (TPP, Techno Plastic Products AG, Trasadingen, Switzerland) until the confluence density was achieved. The culture was carried out (performed) in DMEM culture medium supplemented with 10% of bovine calf serum (Nutricell), 100 UI/mL of penicillin and 100µg/mL of streptomycin sulfate(Nutricell), in a 37°C incubator with humidified atmosphere with 5% CO₂ (Melo et al., 2000, 2001).

The cytotoxicity test was performed in 96 well plates (IWAKI, Asahi Techno Glass, Co., Funabasi, Japan), with 3 x 10⁴ cells/mL in each well, followed by a 48 hour incubation at 37°C period. The initial cellular viability was verified through a Trypan Blue exclusion test. After the incubation period of 48 hours the medium was replaced by a supplemented DMEM medium with different concentrations (up to 1 mg/mL) of the freeze-dried samples, described below:

| **Sample** | **Origin** | **Strains** |
|---|---|---|
| 1 | acquired from third-parties | - |
| 2 | acquired from third-parties | - |
| 3 | Obtained according to the present invention (Example 6) | ATCC 21000 |
| 4 | Obtained according to the present invention (Example 6) | ATCC 21000 |
| 5 | Obtained according to the present invention (Example 6) | T248 |

After 24 hours of treatment, the cultures were processed according to specific test protocols in order to determine acid nucleic content, neutral red incorporation (NR) and MTT reduction (MTT).

The collagenolytic and gelatinolytic proteases obtained from *C. histolyticum* cultures according to the present invention did not demonstrate cytotoxicity, as observed in the results presented in Table 9.

**Table 9. Cytotoxicity test results of collagenolytic and gelatinolytic proteases obtained from C. histolyticum cultures, ATCC 21000 e T248, supernatants produced according to the present invention, and collagenolytic and gelatinolytic proteases present in the raw material acquired from third parties.**

| **Sample** | **Activity (U/g)** | **IC50 (mg/mL)** | | | **IC50 (U/mL)** | | |
|---|---|---|---|---|---|---|---|
| | | **MTT** | **VN** | **nucleic acids** | **MTT** | **VN** | **nucleic acids** |
| 1 | 2214.00 | 0.35 | 0.35 | 0.35 | 0.69 | 0.69 | 0.648 |
| 2 | 2090.00 | 0.33 | 0.31 | 0.31 | 0.69 | 0.69 | 0.648 |
| 3 | 2429.00 | * | 0.90 | 0.90 | 2.43 | 2.43 | 2.186 |
| 4 | 3073.00 | * | * | * | 3.07 | 3.07 | 3.073 |
| 5 | 3646.00 | 0.20 | 0.20 | 0.20 | 0.73 | 0.73 | 0.729 |

## Claims

1. An animal product-free culture medium for bacteria of the genus *Clostridium* comprising water, non-animal origin peptone, or its derivatives, yeast extract and the amino acids cysteine and arginine, or pharmaceutically acceptable salts thereof.

2. The culture medium according to claim 1, wherein the non-animal origin peptone is a vegetable peptone selected from the group consisting of soy, cotton, wheat, sunflower, rice, peanuts, fava bean, peas, potato, corn and mixtures thereof.

3. The culture medium according to claim 2, wherein the vegetable peptone is a soy peptone selected from the group consisting of Hy Soy, Soytone, Amisoy, NZ Soy BL4, NZ Soy BL7, Hy Pep 1510, Hy Pep 1511, , Stedygro Soy SE50M, Proyield Soy, SE50MAF-UF, Soy peptone 312, and mixtures thereof.

4. The culture medium according to claim 1, wherein the yeast extract is selected from the group consisting of Bacto YE, Hy-Pep YE, Hy-Yeast 412, Hy-Yeast 413, Hy-Yeast 502, Hy-Yeast 501, Hy-Yeast 503 Prodex 710 SD, Prodex NS70SD, Pronal 5001, YE 11, YE 151, YE 251, YE 70161, YE Y4250, Synth, YE 207, YE 5114 and mixtures thereof.

5. The culture medium according to claim 1, wherein the bacteria is *Clostridium histolyticum,* the non-animal origin peptone is NZ soy BL4, the yeast extract is YE 251, the cysteine is cysteine hydrochloride and the arginine is arginine hydrochloride.

6. The culture medium according to claim 1, wherein the concentration of non-animal origin peptone ranges from 0.5% to 5% w/v, yeast extract from 0.5% to 5% w/v, cysteine, or its pharmaceutically acceptable salts, from 0.01% to 0.1% w/v, and arginine, or its pharmaceutically acceptable salts, from 0.1% to 1% w/v.

7. Culture medium according to claim 6, wherein the concentration of non-animal origin peptone is 3% w/v, yeast extract is 3% w/v, cysteine, or its pharmaceutically acceptable salts, is 0.0625% w/v and arginine, or its pharmaceutically acceptable salts, is 0.375% w/v.

8. The culture medium according to claim 1, further comprising one or more additives selected from the group consisting of agent for adjustment of pH from 7 to 8, reducing agent, inorganic salts and vitamins.

9. The culture medium according to claim 8, wherein the reducing agent is selected from the group consisting of sodium thioglycolate, sodium bisulphite, iron salts, glucose and mixtures thereof; the inorganic salts are selected from the group consisting of NaCl, KCl, Na2HPO4, NaH2PO4, KH2PO4,K2HPO4, MgSO4, FeSO4, MnSO4 and mixtures thereof; and the vitamins are selected from the group consisting of biotin, pimelic acid, nicotinamide, calcium pantothenate, folic acid, thiamine nitrate, riboflavin, 4-aminobenzoic acid (PABA) and mixtures thereof.

10. Culture medium according to claim 1, which is liquid and sterile.

11. Culture medium according to claim 1, for use in a process for producing *Clostridium histolyticum* liquid culture supernatant comprising one or more proteases with collagenolytic and gelatinolytic activity.

12. A process for producing supernatant of *Clostridium histolyticum* liquid culture comprising one or more proteases with collagenolytic and gelatinolytic activity comprising the following steps:
a) providing a sterile animal product-free culture medium comprising water, vegetable-origin peptone, yeast extract and cysteine or its pharmaceutically acceptable salts;
b) culturing an aliquot of the *Clostridium histolyticum* stock culture in the culture medium in step (a), under anaerobic conditions at about 37°C before reaching the stationary growth phase in order to obtain an inoculum;
c) providing a sterile animal product-free culture medium for *Clostridium* bacteria according to claim 1;
d) adding to the culture medium in step (c) a volume of inoculum from step (b) equal to or lower than 10% of the final volume of the medium defined in (c);
e) culturing the *C. histolyticum* obtained in (d) under anaerobic conditions at about 37°C until the stationary growth phase is attained;
f) removing cellular material and other particulate matter from the liquid phase of the culture in step (e), yielding a supernatant comprising one or more proteases with collagenolytic and gelatinolytic activity.

13. The process according to claim 12, wherein the step (b) comprising at least the following steps: 1) culturing a stock culture in the culture medium from step (a) in a ratio of 0.1:1 v/v for about 16 hours in order to obtain a pre-inoculum; 2) adding to the pre-inoculum obtained in step 1) a culture medium volume from step (a) equal to or higher than the double of the culture medium volume from step 1) and maintaining the culture for about 12 hours in order to obtain an inoculum.

14. The process according to claim 12, wherein the *C. histolyticum* culture during the step (e) involves the addition of one or more agents to adjustment pH in sufficient amount to maintain the culture medium pH from 6.5 to 8.0 and wherein the step (f) is carried out through filtration, centrifugation or both.

15. The process according to claim 12, comprising one or more additional steps for the purification of one or more collagenolytic and gelatinolytic proteases from the supernatant obtained in step (f).

16. The process according to claim 12, wherein the animal product-free culture medium employed in step(a) comprises water, 0.5% to 5% w/v of vegetable peptone, 0.5% to 5% w/v of yeast extract, 0.01% to 0.1% w/v of cysteine, or its pharmaceutically acceptable salts, and pH between 6.5 and 8.0.

17. The process according to claim 16, wherein the culture medium comprises water, 3% w/v of vegetable peptone, 3% w/v of yeast extract, 0.0625% w/v of cysteine, or its pharmaceutically acceptable salts, and pH between 6.5 and 8.0.

18. The process according to claim 12, wherein the animal product-free culture medium employed in step(c) comprises water, 3% w/v of vegetable peptone, 3% w/v of yeast extract, 0.0625% w/v of cysteine, or its pharmaceutically acceptable salts, 0.375% w/v of arginine, or its pharmaceutically acceptable salts, and pH between 7.0 and 8.0.

## Patentansprüche

1. Tierproduktfreies Kulturmedium für Bakterien der Gattung *Clostridium,* das Wasser, Pepton nichttierischen Ursprungs oder Derivate davon, Hefeextrakt und die Aminosäuren Cystein und Arginin oder pharmazeutisch unbedenkliche Salze davon umfasst.

2. Kulturmedium nach Anspruch 1, wobei das Pepton nichttierischen Ursprungs ein pflanzliches Pepton ist, das aus der Gruppe ausgewählt ist, bestehend aus Soja, Baumwolle, Weizen, Sonnenblume, Reis, Erdnüssen, Ackerbohne, Erbsen, Kartoffel, Mais und Mischungen davon.

3. Kulturmedium nach Anspruch 2, wobei das pflanzliche Pepton ein Sojapepton ist, das aus der Gruppe ausgewählt ist, bestehend aus Hy Soy, Soytone, Amisoy, Nz Soy BL4, NZ Soy BL7, Hy Pep 1510, Hy Pep 1511, Stedygro Soy SE50M, Proyield Soy, SE50MAF-UF, Soy peptone 312 und Mischungen davon.

4. Kulturmedium nach Anspruch 1, wobei das Hefeextrakt aus der Gruppe ausgewählt ist, bestehend aus Bacto YE, Hy-Pep YE, Hy-Yeast 412, Hy-Yeast 413, Hy-Yeast 502, Hy-Yeast 501, Hy-Yeast 501, Hy-Yeast 503 Prodex 710 SD, Prodex NS70SD, Pronal 5001, YE 11, YE 151, YE 251, YE 70161, YE Y4250, Synth, YE 207, YE 5114 und Mischungen davon.

5. Kulturmedium nach Anspruch 1, wobei die Bakterien *Clostridium histolyticum* sind, das Pepton nichttierischen Ursprungs NZ soy BL4 ist, das Hefeextrakt YE 251 ist, das Cystein Cysteinhydrochlorid ist und das Arginin Argininhydrochlorid ist.

6. Kulturmedium nach Anspruch 1, wobei die Konzentration von Pepton nichttierischen Ursprungs im Bereich von 0,5 bis 5 % Gew./Vol. liegt, von Hefeextrakt im Bereich von 0,5 bis 5 % Gew./Vol. liegt, von Cystein oder pharmazeutisch unbedenklichen Salzen davon im Bereich von 0,01 bis 0,1 % Gew./Vol. liegt und von Arginin oder pharmazeutisch unbedenklichen Salzen davon im Bereich von 0,1 bis 1 % Gew./Vol. liegt.

7. Kulturmedium nach Anspruch 6, wobei die Konzentration von Pepton nichttierischen Ursprungs 3 % Gew./Vol. beträgt, von Hefeextrakt 3 % Gew./Vol. beträgt, von Cystein oder pharmazeutisch unbedenklichen Salzen davon 0,0625 % Gew./Vol. beträgt und von Arginin oder pharmazeutisch unbedenklichen Salzen davon 0,375 % Gew./Vol. beträgt.

8. Kulturmedium nach Anspruch 1, das ferner einen oder mehrere Zusatzstoffe umfasst, die aus der Gruppe ausgewählt sind, bestehend aus einem M ittel zum Anpassen des pH-Werts auf 7 bis 8, einem Reduktionsmittel, anorganischen Salzen und Vitaminen.

9. Kulturmedium nach Anspruch 8, wobei das Reduktionsmittel aus der Gruppe ausgewählt ist, bestehend aus Natriumthioglycolat, Natriumbisulfit, Eisensalzen, Glucose und Mischungen davon; die anorganischen Salze aus der Gruppe ausgewählt sind, bestehend aus NaCl, KCl, Na2HPO4, NaH2PO4, KH2PO4, K2HPO4, MgSO4, FeSO4, MnSO4 und Mischungen davon; und die Vitamine aus der Gruppe ausgewählt sind, bestehend aus Biotin, Pimelinsäure, Nikotinamid, Calciumpantothenat, Folsäure, Thiaminnitrat, Riboflavin, 4-Aminobenzoesäure (PABA) und Mischungen davon.

10. Kulturmedium nach Anspruch 1, das flüssig und steril ist.

11. Kulturmedium nach Anspruch 1 zur Verwendung in einem Verfahren zum Produzieren eines *Clostridiumhistolyticum*-Flüssigkulturüberstands, der eine oder mehrere Proteasen mit kollagenolytischer und gelatinolytischer Aktivität umfasst.

12. Verfahren zum Produzieren eines *Clostridiumhistolyticum*-Flüssigkulturüberstands, der eine oder mehrere Proteasen mit kollagenolytischer und gelatinolytischer Aktivität umfasst, das die folgenden Schritte umfasst:
a) Bereitstellen eines sterilen tierproduktfreien Kulturmediums, das Wasser, Pepton pflanzlichen Ursprungs, Hefeextrakt und Cystein oder pharmazeutisch unbedenkliche Salze davon umfasst;
b) Kultivieren einer Teilprobe der *Clostridiumhistolyticum*-Stammkultur im Kulturmedium in Schritt (a) unter anaeroben Bedingungen bei ungefähr 37 °C, bevor die stationäre Wachstumsphase erreicht wird, um ein Inokulum zu erhalten;
c) Bereitstellen eines sterilen tierproduktfreien Kulturmediums für *Clostridium*-Bakterien nach Anspruch 1;
d) Hinzufügen eines Volumens des Inokulums von Schritt (b) zum Kulturmedium in Schritt (c), das kleiner gleich 10 % des Endvolumens des in (c) definierten Mediums ist;
e) Kultivieren des *C. histolyticum,* das in (d) erhalten wird, unter anaeroben Bedingungen bei ungefähr 37 °C, bis die stationäre Wachstumsphase erreicht ist;
f) Entfernen von Zellmaterial und anderer partikelförmiger Materie aus der Flüssigphase der Kultur in Schritt (e), wobei ein Überstand erhalten wird, der eine oder mehrere Proteasen mit kollagenolytischer und gelatinolytischer Aktivität umfasst.

13. Verfahren nach Anspruch 12, wobei Schritt (b) zumindest die folgenden Schritte umfasst: 1) Kultivieren einer Stammkultur im Kulturmedium aus Schritt (a) in einem Verhältnis von 0,1:1 Vol./Vol. für ungefähr 16 Stunden, um ein Präinokulum zu erhalten; 2) Hinzufügen eines Kulturmediumvolumens aus Schritt (a) zu dem in Schritt 1) erhaltenen Präinokulum, das größer gleich dem Doppelten des Kulturmediumvolumens von Schritt 1) ist, und Halten der Kultur für ungefähr 12 Stunden, um ein Inokulum zu erhalten.

14. Verfahren nach Anspruch 12, wobei die *C.-histolyticum-*Kultur während Schritt (e) das Hinzufügen eines oder mehrerer Mittel zum Einstellen des pH-Werts in einer Menge umfasst, die ausreichend ist, um den pH-Wert des Kulturmediums auf 6,5 bis 8,0 zu halten, und wobei Schritt (f) durch Filtration, Zentrifugation oder beides durchgeführt wird.

15. Verfahren nach Anspruch 12, das ein oder mehrere zusätzliche Schritte zum Reinigen einer oder mehrerer kollagenolytischer und gelatinolytischer Proteasen aus dem in Schritt (f) erhaltenen Überstand umfasst.

16. Verfahren nach Anspruch 12, wobei das in Schritt (a) verwendete tierproduktfreie Kulturmedium Wasser, 0,5 bis 5 % Gew./Vol. pflanzliches Pepton, 0,5 bis 5 % Gew./Vol. Hefeextrakt, 0,01 bis 0,1 % Gew.-/Vol. Cystein oder pharmazeutisch unbedenkliche Salze davon umfasst und einen pH-Wert zwischen 6,5 und 8,0 aufweist.

17. Verfahren nach Anspruch 16, wobei das Kulturmedium Wasser, 3 % Gew.-/Vol. pflanzliches Pepton, 3 % Gew.-/Vol. Hefeextrakt, 0,0625 % Gew.-/Vol. Cystein oder pharmazeutisch unbedenkliche Salze davon umfasst und einen pH-Wert zwischen 6,5 und 8,0 aufweist.

18. Verfahren nach Anspruch 12, wobei das in Schritt (c) verwendete tierproduktfreie Kulturmedium Wasser, 3 % Gew.-/Vol. pflanzliches Pepton, 3 % Gew.-/Vol. Hefeextrakt, 0,0625 % Gew.-/Vol. Cystein oder pharmazeutisch unbedenkliche Salze davon, 0,375 % Gew./Vol Arginin oder pharmazeutisch unbedenkliche Salze davon umfasst und einen pH-Wert zwischen 7,0 und 8,0 aufweist.

## Revendications

1. Milieu de culture exempt de produits animaux pour des bactéries du genre *Clostridium* comprenant de l'eau, une peptone d'origine non animale, ou ses dérivés, un extrait de levure et les acides aminés cystéine et arginine, ou des sels de ceux-ci pharmaceutiquement acceptables.

2. Milieu de culture selon la revendication 1, dans lequel la peptone d'origine non animale est une peptone végétale sélectionnée dans le groupe consistant en le soja, le coton, le blé, le tournesol, le riz, la cacahuète, la fève, le pois, la pomme de terre, le maïs et des mélanges de celles-ci.

3. Milieu de culture selon la revendication 2, dans lequel la peptone végétale est une peptone de soja sélectionnée dans le groupe consistant en Hy Soy, Soytone, Amisoy, NZ Soy BL4, NZ Soy BL7, Hy Pep 1510, Hy Pep 1511, Stedygro Soy SE50M, Proyield Soy, SE50MAF-UF, Soy peptone 312, et des mélanges de celles-ci.

4. Milieu de culture selon la revendication 1, dans lequel l'extrait de levure est sélectionné dans le groupe consistant en le Bacto YE, Hy-Pep YE, Hy-Yeast 412, Hy-Yeast 413, Hy-Yeast 502, Hy-Yeast 501, Hy-Yeast 503, Prodex 710 SD, Prodex NS70SD, Pronal 5001, YE 11, YE 151, YE 251, YE 70161, YE Y4250, Synth, YE 207, YE 5114 et des mélanges de ceux-ci.

5. Milieu de culture selon la revendication 1, dans lequel la bactérie est *Clostridium histolyticum,* la peptone d'origine non animale est NZ soy BL4, l'extrait de levure est YE 251, la cystéine est le chlorhydrate de cystéine et l'arginine est le chlorhydrate d'arginine.

6. Milieu de culture selon la revendication 1, dans lequel la concentration de la peptone d'origine non animale est comprise entre 0,5 % et 5 % p/v, l'extrait de levure entre 0,5 % et 5 % p/v, la cystéine, ou ses sels pharmaceutiquement acceptables, entre 0,01 % et 0,1 % p/v, et l'arginine, ou ses sels pharmaceutiquement acceptables, entre 0,1 % et 1 % p/v.

7. Milieu de culture selon la revendication 6, dans lequel la concentration de la peptone d'origine non animale est 3 % p/v, l'extrait de levure est 3 % p/v, la cystéine, ou ses sels pharmaceutiquement acceptables, est 0,0625 % p/v, et l'arginine, ou ses sels pharmaceutiquement acceptables, est 0,375 % p/v.

8. Milieu de culture selon la revendication 1, comprenant en outre un ou plusieurs additifs sélectionnés dans le groupe consistant en un agent pour l'ajustement du pH entre 7 et 8, un agent réducteur, des sels inorganiques et des vitamines.

9. Milieu de culture selon la revendication 8, dans lequel l'agent réducteur est sélectionné dans le groupe consistant en le thioglycolate de sodium, le bisulfite de sodium, des sels de fer, le glucose et des mélanges de ceux-ci ; les sels inorganiques sont sélectionnés dans le groupe consistant en le NaCl, KCl, Na2HPO4, NaH2PO4, KH2PO4, K2HPO4, MgSO4, FeSO4, MnSO4 et des mélanges de ceux-ci ; et les vitamines sont sélectionnées dans le groupe consistant en la biotine, l'acide pimélique, le nicotinamide, le pantothénate de calcium, l'acide folique, le nitrate de thiamine, la riboflavine, l'acide 4-aminobenzoïque (PABA) et des mélanges de ceux-ci.

10. Milieu de culture selon la revendication 1, qui est liquide et stérile.

11. Milieu de culture selon la revendication 1, destiné à être utilisé dans un procédé de production d'un surnageant d'une culture liquide de *Clostridium histolyticum* comprenant une ou plusieurs protéases possédant une activité collagénolytique et gélatinolytique.

12. Procédé de production d'un surnageant d'une culture liquide de *Clostridium histolyticum* comprenant une ou plusieurs protéases possédant une activité collagénolytique et gélatinolytique, comprenant les étapes suivantes consistant à :
a) mettre à disposition un milieu de culture stérile exempt de produits animaux comprenant de l'eau, une peptone d'origine végétale, un extrait de levure et de la cystéine ou ses sels pharmaceutiquement acceptables ;
b) mettre en culture une aliquote d'une culture stock de *Clostridium histolyticum* dans le milieu de culture de l'étape (a), dans des conditions anaérobies à environ 37 °C avant d'atteindre la phase de croissance stationnaire afin d'obtenir un inoculum ;
c) mettre à disposition un milieu de culture stérile exempt de produits animaux pour des bactéries *Clostridium* selon la revendication 1 ;
d) ajouter au milieu de culture de l'étape (c) un volume d'inoculum de l'étape (b) inférieur ou égal à 10 % du volume final du milieu défini en (c) ;
e) mettre en culture la *C. histolyticum* obtenue en (d) dans des conditions anaérobies à environ 37 °C jusqu'à atteindre la phase de croissance stationnaire ;
f) éliminer le matériel cellulaire et les autres matières particulaires de la phase liquide de la culture de l'étape (e), en produisant un surnageant comprenant une ou plusieurs protéases possédant une activité collagénolytique et gélatinolytique.

13. Procédé selon la revendication 12, dans lequel l'étape (b) comprend au moins les étapes suivantes consistant à : 1) mettre en culture une culture stock dans le milieu de culture de l'étape (a) selon un rapport de 0,1:1 v/v pendant environ 16 heures afin d'obtenir un pré-inoculum ; 2) ajouter au pré-inoculum obtenu à l'étape 1) un volume de milieu de culture de l'étape (a) supérieur ou égal au double du volume de milieu de culture de l'étape 1) et maintenir la culture pendant environ 12 heures afin d'obtenir un inoculum.

14. Procédé selon la revendication 12, dans lequel la culture de *C. histolyticum* pendant l'étape (e) implique l'ajout d'un ou de plusieurs agents d'ajustement du pH en une quantité suffisante pour maintenir le pH du milieu de culture entre 6,5 et 8,0, et dans lequel l'étape (f) est réalisée par une filtration, centrifugation, ou les deux.

15. Procédé selon la revendication 12, comprenant une ou plusieurs étapes supplémentaires pour la purification d'une ou de plusieurs protéases collagénolytiques et gélatinolytiques à partir du surnageant obtenu à l'étape (f).

16. Procédé selon la revendication 12, dans lequel le milieu de culture exempt de produits animaux employé à l'étape (a) comprend de l'eau, 0,5 % à 5 % p/v de peptone végétale, 0,5 % à 5 % p/v d'extrait de levure, 0,01 % à 0,1 % p/v de cystéine, ou ses sels pharmaceutiquement acceptables, et un pH compris entre 6,5 et 8,0.

17. Procédé selon la revendication 16, dans lequel le milieu de culture comprend de l'eau, 3 % p/v de peptone végétale, 3 % p/v d'extrait de levure, 0,0625 % p/v de cystéine, ou ses sels pharmaceutiquement acceptables, et un pH compris entre 6,5 et 8,0.

18. Procédé selon la revendication 12, dans lequel le milieu de culture exempt de produits animaux employé à l'étape (c) comprend de l'eau, 3 % p/v de peptone végétale, 3 % p/v d'extrait de levure, 0,0625 % p/v de cystéine, ou ses sels pharmaceutiquement acceptables, 0,375 % p/v d'arginine, ou ses sels pharmaceutiquement acceptables, et un pH compris entre 7,0 et 8,0.
